(19) Europäisches Patentamt
European Patent Office
Office européen des brevets

(11) EP 1 141 056 B1

(12) EUROPEAN PATENT SPECIFICATION

(45) Date of publication and mention of the grant of the patent:
**25.08.2010 Bulletin 2010/34**

(21) Application number: **99967580.4**

(22) Date of filing: **22.12.1999**

(51) Int Cl.:
***C08F 265/04*** *(2006.01)* ***C08F 291/00*** *(2006.01)*

(86) International application number:
**PCT/US1999/030790**

(87) International publication number:
**WO 2000/040628 (13.07.2000 Gazette 2000/28)**

(54) **BRANCHED/BLOCK COPOLYMERS FOR TREATMENT OF KERATINOUS SUBSTRATES**

VERZWEIGTE BLOCKCOPOLYMERE FÜR DIE BEHANDLUNG DER OBERFLÄCHE DES KERATINS

COPOLYMERES RAMIFIES/SEQUENCES PERMETTANT DE TRAITER DES SUBSTRATS KERATINIQUES

(84) Designated Contracting States:
**AT BE CH CY DE DK ES FI FR GB GR IE IT LI LU MC NL PT SE**

(30) Priority: **30.12.1998 US 223664**

(43) Date of publication of application:
**10.10.2001 Bulletin 2001/41**

(73) Proprietor: **Lubrizol Advanced Materials, Inc.**
**Cleveland, OH 44141-3247 (US)**

(72) Inventors:
- **GALLEGUILLOS, Ramiro**
  **Hudson, OH 44236 (US)**
- **SMITH, David, J.**
  **Amherst, OH 44001 (US)**
- **CONSTANTINO, Steven, P.**
  **LaGrange, OH 44050 (US)**

(74) Representative: **von Kreisler Selting Werner**
**Deichmannhaus am Dom**
**Bahnhofsvorplatz 1**
**50667 Köln (DE)**

(56) References cited:
**EP-A- 0 233 014** **EP-A- 0 398 576**
**WO-A1-97/09030** **WO-A1-97/09031**
**GB-A- 1 484 053** **US-A- 4 085 167**
**US-A- 4 130 517** **US-A- 5 225 456**
**US-A- 5 403 894**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).



Printed by Jouve, 75001 PARIS (FR)

## Description

### Background of the Invention

The present invention is directed to polymers containing a branched/block copolymer structure, which is useful for treatment of keratinous substrates, especially to cosmetic compositions, such as hair sprays, hair conditioners, hair setting lotions, creams, and the like, which incorporate the polymers. The polymers provide the cosmetic compositions with greater holding power, less flaking, and better ability to stylize than conventional polymers used in hair and similar cosmetic preparations.

Both natural and synthetic polymers, usually incorporated into an aqueous or an aqueous/alcoholic solution, are in current use as hair lacquers, hair-setting lotions, and the like. The function of such polymers is to impart "body" and holding power to the hair.

At the present time, the principal polymers or polymers employed in hair sprays, setting lotions, and hair conditioners include polyvinyl pyrrolidone (PVP) homopolymers and copolymers, half esters of polyvinyl ethers-maleic anhydride, polyvinyl acetate-crotonic acid co- and terpolymers, half esters of ethylene-maleic anhydride, acrylates and others.

With the exception of vinyl pyrrolidone homopolymers, conventionally employed synthetic polymers and polymers used in hair sprays, and the like, tend to impart excessive stiffness to the hair, causing an unnatural look. In addition, incorporation of such synthetic polymers or polymers into hair care compositions sometimes leads to excessive flaking, thereby making the compositions unsatisfactory from a commercial standpoint.

Although polyvinyl pyrrolidone homopolymers and copolymers provide a more natural look in that they are free from some of the disadvantages of other commercially available products, they tend to provide less satisfactory holding of the hair at high humidity levels.

Typical hair styling polymers are random copolymers which are prepared by polymerizing two or more hydrophilic, anionic, cationic, or hydrophobic monomers, such as acrylic or vinyl monomers. The backbone of the resulting polymer is typically composed of a statistically random distribution of all the monomers. The ratio of these monomers is selected in such a way as to obtain a resin with a certain hydrophilic and hydrophobic balance.

The hydrophobic monomers generally provide better hairstyle retention at high humidity levels. Polymers, in which hydrophobic monomers predominate heavily, however, have poor solubility in water-ethanol mixtures, are not readily washable from the hair, and tend to flake and feel plastic-like on the hair. They are therefore unsuited to use in hair styling formulations.

On the other hand, polymers with high levels of hydrophilic groups have good solubility in water/ethanol mixtures and are washable from the hair. However, they are generally too sensitive to moisture, becoming tacky, and therefore do not hold a hairstyle under conditions of high humidity. U.S.-A- 3,954,960 to Valan, for example, discloses a random copolymer of purely hydrophilic monomers. This is a film forming resin of a quaternized copolymer of vinyl pyrrolidone and a copolymerizable vinyl monomer such as a di-lower alkyl alkyaminoalkyl (or hydroxy alkyl) acrylate or methacrylate. Quatemized polymers of polyvinyl pyrrolidone, however, tend to be highly moisture sensitive and have overall poor performance in hairstyle retention and tack.

Accordingly, it is desirable to build a resin with a balance of hydrophilic and hydrophobic groups to achieve a combination of performance properties, such as style retention at high humidity, tack, hardness, flaking, washability from the hair, and other subjective performance attributes.

A typical example of a random copolymer having a hydrophilic/hydrophobic balance is disclosed in U.S.-A -3,914,403 to Valan. Valan discloses a film-forming resin formed from polyvinyl pyrrolidone, vinyl acetate, and a cationic monomer. The polyvinyl pyrrolidone and the cationic groups form the hydrophilic portion of the resin, while vinyl acetate provides the hydrophobic portion. By varying the ratio of polyvinyl pyrrolidone to vinyl acetate, water soluble or water insoluble polymers are obtained. The highly water soluble polymers tend to have poor hair style retention at high humidity, whereas the highly insoluble ones are likely to be un-washable and too plastic-like.

U.S.-A- 3,925,542 to Viout, et al. and U.S.-A- 5,196,495 to Chuang, et al. disclose additional examples of random copolymers with various hydrophilic/hydrophobic balances. Uses for the copolymers include aerosols, lacquers, non-aerosol hair sprays, hair setting creams, and setting lotions.

U.S.-A- 4,007,005 to Patel discloses a hair setting resin based on a random copolymer of a reactive polyamide epichlorohydrin and polyvinyl pyrrolidone. The copolymer provides long style retention at high humidity. However, the reactive polymers are toxic and lack washability from the hair when used as aerosols, non-aerosol hair sprays and setting lotions.

Due to environmental regulations controlling the emission of volatile organic compounds (VOC's) into the atmosphere, VOC emissions have been restricted to 80% by weight of the hair styling formulation in some states, with further restrictions to 55% anticipated. To meet the regulations, reduced VOC hair styling formulations are being developed. Water is substituted for part or all of the organic solvents conventionally used in such formulations. U.S.-A-

5,565,193 to Midha, et al. discloses a random copolymer hairstyling resin primarily formed from monomers such as n-butyl acrylate (the hydrophobic component), and acrylic acid, the (hydrophilic component), grafted with siloxane to balance the properties and to render the resin suitable for formulation in an 80% VOC composition. However, such polymers tend to become too soft and produce negative beading on the hair in low VOC formulations.

U.S.-A- 5.620.683 to Tong, et al. discloses a resin comprising a random copolymer of n-alkyl acrylamide (the hydrophobic component) and acrylic acid (the hydrophilic component). Although the polymers are said to be suited to use in low VOC formulations, such polymers tend to be insoluble in water. Rather, they form a slurry in water and ethanol blends. Only upon adding the liquefied propellant gas, such as dimethyl ether, to the aerosol cans, does the resin dissolve. Preparing a slurry and pumping it into the aerosol cans is impractical for most purposes. In addition, because the resin is insoluble in water, it may prove difficult to wash from the hair.

U.S.-A- 5,599,524 to Morawski, et al. discloses a hair spray composition in a formulation having 80% VOC's, or less. A defoaming agent is added to a conventional hair resin to reduce surface tension and to eliminate foaming of aerosol and non-aerosol hair sprays. The composition does not provide an improvement in flaking, fly away, or humidity resistance over conventional formulations.

U.S.-A-.5,501,851 to Mudge, et al. discloses a random copolymer of butyl acrylate, methyl methacrylate, hydroxyethyl acrylate, and methacrylic acid for low VOC formulations. The polymer is dispersed in an emulsion to render it later removable with a shampoo.

GB-A-1 484 053 discloses a curable resin composition for the treatment of a paper web. The curable resin contains a blend of several different polymer components. The polymers are made in a one step polymerization process to yield a non-blocky copolymer product as evidenced by a single glass transition temperature.

US-A-5 225 456 is directed to a polymer coating for PVC substrates. The disclosed polymers are essentially composed of aliphatic and aromatic esters of (meth)acrylic acid and hence are hydrophobic.

US-A-5 403 894 discloses a core-shell polymer useful as an acrylic modifier for cement.

EP-A-0 398 576 describes multi-stage polymer particles which find use as thickeners. One stage of the multi-stage polymer particle contains a hydrophobically modified, ionically soluble polymer stage which is polymerized from a hydrophobic monomer, an ethylenically unsaturated ionizable monomer, an ethylenically unsaturated non-ionic monomer, and optionally, a multi-functional compound. The disclosed multi-stage polymer particles are not block copolymers but simply core-shell particles. These polymers are not film forming but are thickeners.

US-A-4 130 517 discloses AB type copolymers wherein A is a hydrophobic polymer block and B is a hydrophilic block. The block copolymers are linear block copolymers. The acrylate and methacrylate blocks are made by ionic polymerization.

WO-A-97/09030 and WO-A-97/09031 concern a latex composition suitable for use in shampoo compositions.

The present invention provides for improved block/branched copolymers and hair treatment compositions incorporating the copolymers, which overcome the above-referenced problems, and others.

## Summary of the Invention

The present invention has resulted from the discovery that a block copolymer for use in hair styling compositions can be prepared from ethylenically unsaturated monomers utilizing a polyfunctional organic monomer, having at least two functional groups, or chain extender monomer, where the reactivity of the functional groups is substantially different to produce an AB block copolymer. The polyfunctional monomer polymerizes with a first monomer or mixture of monomers through the functional group having the greater reactivity to form a first or A block. A second monomer or mixture of monomers contains at least one carboxylic acid group and copolymerizes with the less reactive functional group of the chain extender monomer to form a second, or B, block. The result is a block copolymer in which the A-block is more hydrophobic than the B-block so that the copolymer has both hydrophobic and hydrophilic blocks and has a plurality of glass transition temperatures and which provides exceptional utility as a hair styling composition.

In particular, the present invention relates to a film forming block copolymer formed in the presence of a free radical initiator from:

- a polyfunctional monomer having two functional groups, the reactivity of one functional group being higher than that of other functional group;
- first ethylenically unsaturated monomers which copolymerize preferentially with the functional group of the difunctional monomer which has a higher reactivity to form a first block; and
- a second ethylenically unsaturated monomer or monomers, which contains at least one carboxylic acid group and copolymerizes with the functional group of the difunctional monomer which has the lesser reactivity to form a second block, wherein the first block is more hydrophobic than the second block, and the copolymer has both hydrophobic and hydrophilic blocks, which have at least two different glass transition temperatures, wherein
  the polyfunctional monomer is allyl methacrylate, the first ethylenically unsaturated monomers is a mixture of n-butyl

acrylate and (meth)acrylic acid and the second ethylenically unsaturated monomer(s) is selected from the group consisting of acrylic acid, methacrylic acid and mixtures thereof.

Another embodiment of the present invention relates to a hairstyling composition including 0.01 to 20 % by weight of said film forming branched block copolymer.

In accordance with another aspect of the present invention, a hair styling composition includes 1 to 20 weight percent of a block copolymer in accordance with the present invention, together with 20 to 99 weight percent of water and 0 to 80 weight percent of an organic solvent. In addition, a method of preparing a hair styling composition is provided. The method includes preparing an AB block copolymer having hydrophobic and hydrophilic blocks by polymerizing a polyfunctional organic monomer(s) which has at least two functional groups with a first ethylenically unsaturated monomer (s) to form an A-Block, and then polymerizing a second ethylenically unsaturated monomer(s) containing at least one carboxylic acid group with the A-block to form a B-block and a copolymer having hydrophobic and hydrophilic blocks. To prepare hair styling compositions 1-20 % wt. of the block copolymer is combined with 20-97 % wt. of water, 0 to 80 % wt. of organic solvent, 0 to 5 % wt. surfactant and conditioning agents, 0 to 1% wt. fragrance, and other ancillary agents.

Preferred embodiments of the invention are appeared from the dependent claims.

One advantage of the present invention is that it enables hair styling compositions to be prepared with optimal performance properties, such as style retention at high humidity, tack, hardness, flaking, washability from the hair and other subjective performance attributes.

Another advantage of the present invention is that the copolymers provided can be incorporated into hydro-alcoholic hair styling formulations to meet reduced VOC, regulations and they are effective as styling agents in a wide variety of hair styling formulations, including aerosol sprays, mousses, spritzes, gels, setting lotions, and the like.

Yet another advantage of the present invention is that the copolymers can be used for a variety of other applications where a coating composition or film forming polymer would be employed which can benefit from the fact that the block copolymer has hydrophobic and hydrophilic blocks.

## Detailed Description of the Preferred Embodiments

A-Block/branched copolymer having two or more distinct glass transition temperatures ($T_g$) can be tailored to provide the desired properties in a hair care composition. The copolymer has a block structure, consisting essentially of a hard, hydrophilic block, which contributes to a high $T_g$, and a soft, more hydrophobic block, which contributes to a low $T_g$. The hydrophobic block forms the A-Block of the copolymer, while the hydrophilic block forms the B-Block. The hydrophilic B-Block and hydrophobic A-Block contribute different properties to the overall copolymer. The soft, low-$T_g$ hydrophobic A-Block contributes properties such as the formation of a uniform, clear film on the surface of the hair, providing high humidity resistance for durable hair style retention, conditioning and detangling the hair while wet, conferring the dried hair with a soft feel to the touch, adherence to the hair without flaking, reshaping of the hair by application of a curling iron. The hard, high-$T_g$, hydrophilic block provides the copolymer with benefits such as ease of dispersion of the copolymer in water, alcohol, or mixtures thereof, provision of a firm hold when applied to the hair, ease of washability from the hair, and detangling of the wet hair with a comb. These properties can be tailored by varying the composition and length of the blocks. For hair styling and fixing compositions, the hydrophobic A-Block is preferably a polyacrylate, while the hydrophilic branches are preferably formed from methacrylic acids or other polymer-forming carboxylic acids.

The general structure of the copolymer mentioned above can be represented by the following two exemplary structures:

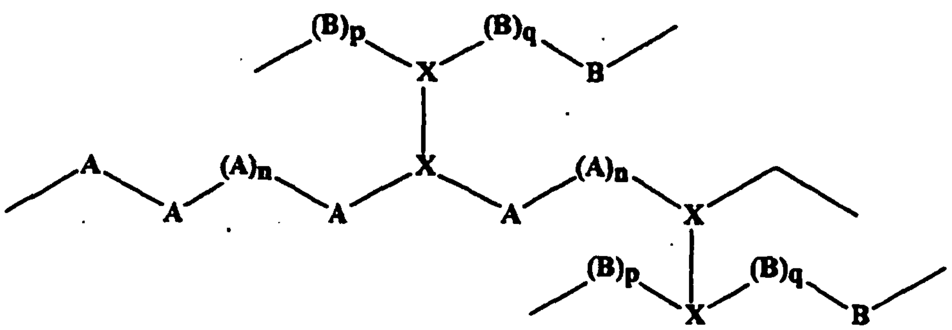

Structure 1.

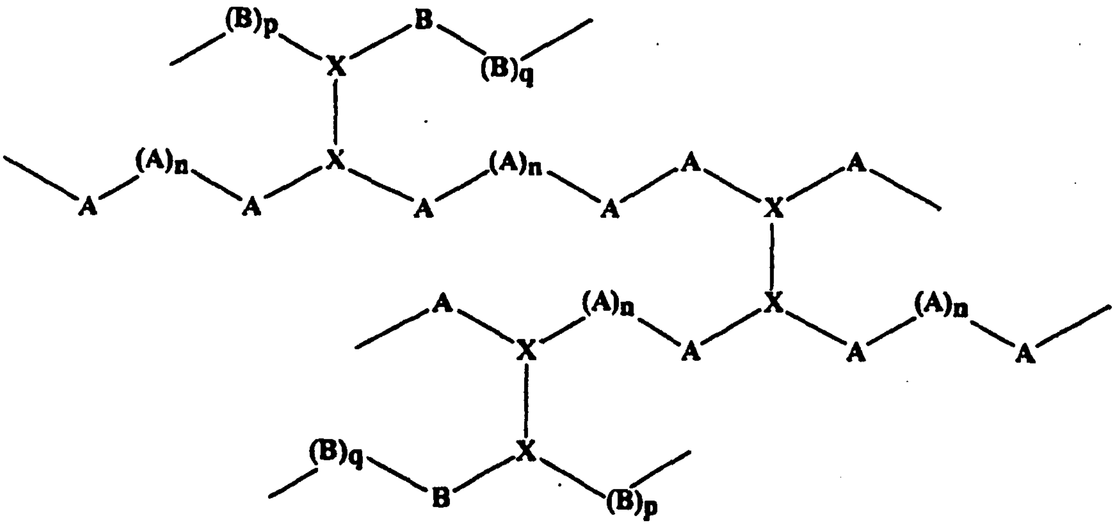

Structure 2.

where A represents the monomer or monomers of the first block, referred to herein as the "A-Block," and B represents the monomer or monomers of the second block, referred to herein as the "B-Block." X represents a chain branching agent or a multifunctional monomer used to link the A and B-Blocks. In these structures, n represents the degree of polymerization of the A-Block, i.e., the number of monomer units in the A-Block.Its value is typically larger than 100. The letters q and p represent the degree of polymerization of the B-Block, i.e. the number of monomer units in the B-Block. Their added value is typically larger than 100. Either q or p can take the value of zero but not both at the same time. The straight line between two monomers (A-A) represents a covalent chemical bond.

The copolymers of this invention are blocky and may form three-dimensional networks. The existence of the two blocks was confirmed by conducting differential scanning calorimetry on dry polymer samples. It is well known that the presence of two or more transition temperatures, $T_g$'s, is a clear indication of the blocky character of the copolymers, see, e.g., "Contemporary Polymer Chemistry" 2nd. Edition by H. Allcock and F. Lampe, Ch. 17, Prentice Hall Publishers, 1990. The A-Blocks and the B-Blocks are covalently or chemically attached through the chain branching agent X.

The average molecular weight of the copolymer can reach up to 1,000,000. The preferred molecular weight is in the 20,000 to 250,000 range. The preferred molecular weight of the A-Block is in the range of 10,000 to 150,000, whereas the preferred molecular weight of the B-Block is in the range of 1,000 to 50,000.

Therefore. the copolymers of this invention attain their unique hair styling and fixing properties attributes due to a combination of soft and hard blocks. The A-Block is a soft more hydrophobic block, with low $T_g$, and the B-Block is a hard, hydrophilic high $T_g$ block. In addition, the length and composition of the blocks of the polymer can be varied to improve specific performance needs. In particular, the copolymers of this invention are designed to provide long lasting hair style retention at high humidity, natural feel, good hair combing, reduced flaking, no build up, and good hair styling and restyling. They are good film formers, water and alcohol soluble or dispersible and washable with water and shampoo.

To form the copolymer of the present invention, a two-step reaction process is used. This polymerization can be performed in a single reactor without having to isolate either the A or B-Block as an intermediate. The first step yields the A-Block portion of the copolymer, while the second step adds on the B-Blocks to form the resulting copolymer. In the first step, the monomers A, i.e., a mixture of n-butyl acrylate and (meth) acrylic acid is copolymerized with a relatively small amount of the second chain extender monomer X. The monomer X has two or more polymerizable functional groups. The reactivity of the functional groups is such that the first monomer A reacts preferentially with a first functional group leaving the second functional group predominately unreacted. Monomer X has both allyl and acrylate and methacrylate groups, and is allyl methacrylate. The acrylate and methacrylate groups polymerize faster, due to its higher reactivity relative to the allyl groups. In the first step, the allyl groups remain predominantly unreacted.

The first and second monomers react to produce a polymer. The polymer may be a linear or a branched polyacrylate with allyl functional side arms.

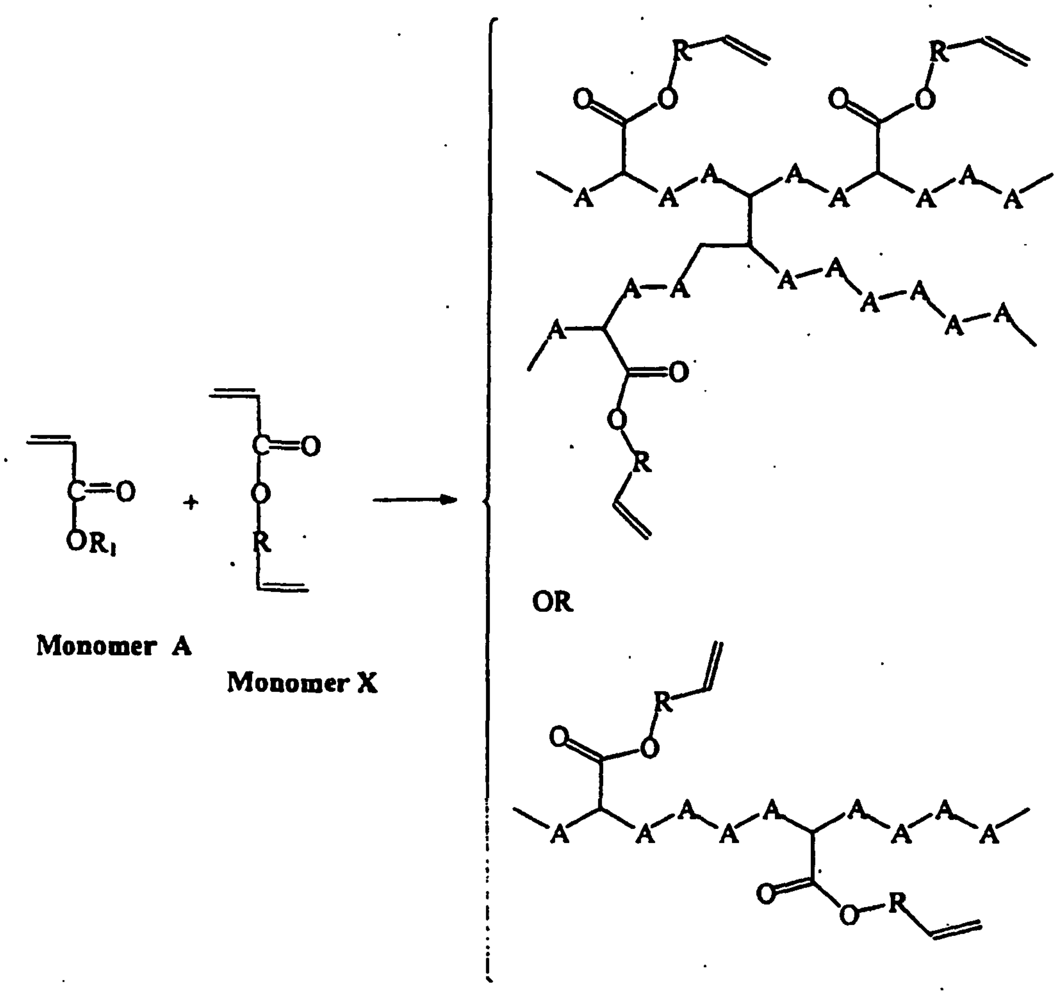

Monomer A

Monomer X

OR

Polymer

where R and $R_1$ are chemical groups described later and where A represents the incorporated monomer A. While two structures for the polymer have been shown, it should be understood that combinations of the two structures, including linear and branched portions, may be formed.

In the second step, monomers B i.e., acrylic acid, methacrylic acid or a mixture thereof is added and reacted with the slower reacting, second functional groups of the polymer to obtain a three-dimensional branched and blocky copolymer.

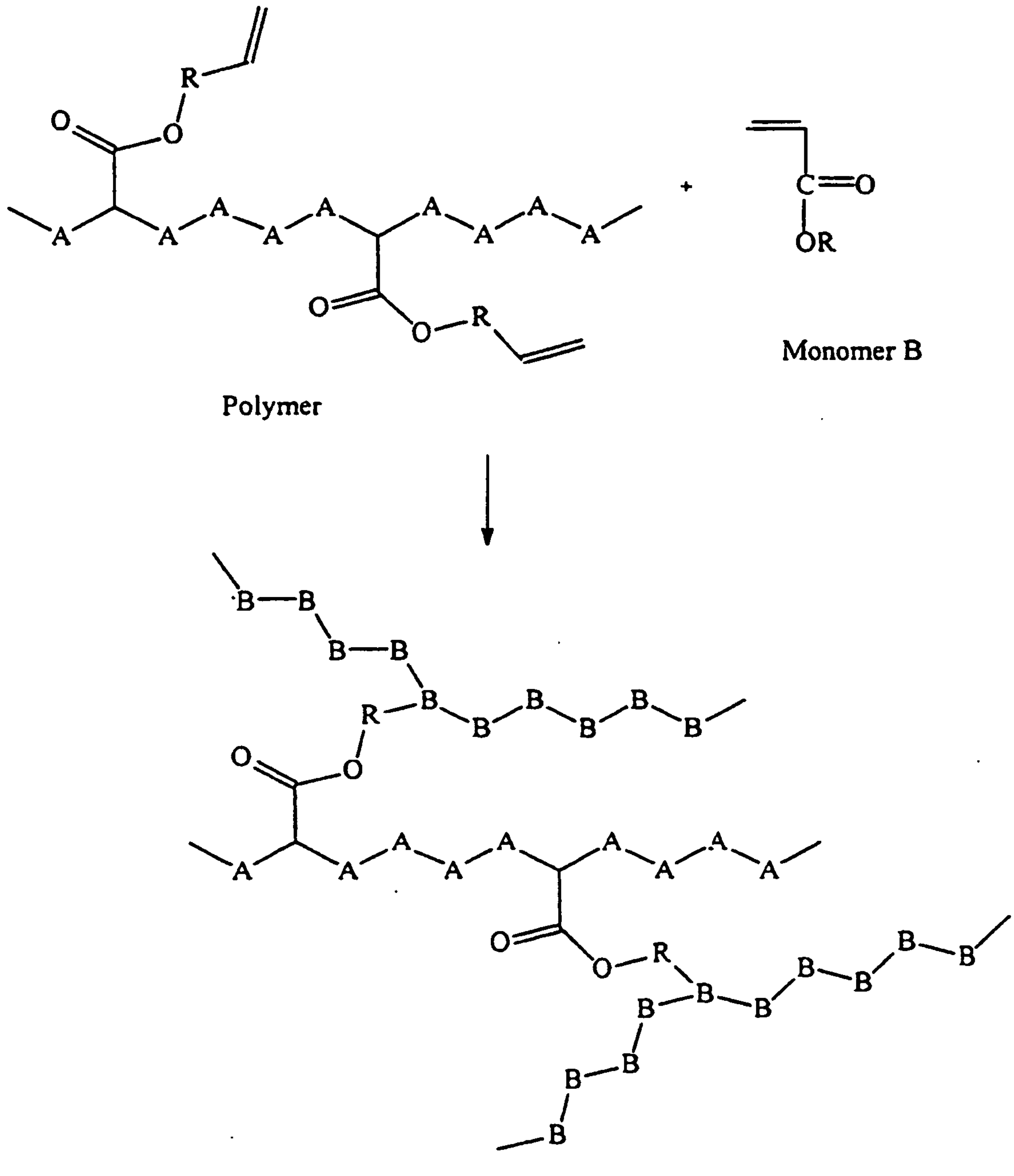

Polymer

Monomer B

Branched Block Copolymer

where R2 is preferably an alkyl group and B represents the incorporated monomer B. The copolymer thus has a backbone, primarily derived from the monomers A and branches derived primarily from the monomer B.

This method is not limited to preparing the hydrophobic monomer Block first with the multifunctional monomer, then preparing the hydrophilic B-Block. The order of addition can be changed. This is obvious to those familiar with the art of polymerization.

The B-Block of the copolymer is a hydrophilic block, while the A-Block is more hydrophobic than the B-Block. The hydrophobicity of the A-Block can be taylored to suit specific performance by incorporating hydrophilic monomers such as where the hydrophilic monomers is less than 60 % mol.

Suitable hydrophobic monomers A include those which are a) water insoluble, that is, less than 0.2 weight percent of the hydrophobic monomer will dissolve in one hundred weight parts water, and b) are ethylenically unsaturated compounds.

The hydrophobic monomer A is a mixture of n-butyl acrylate and (meth)acrylic acid.

The chain branching monomers X used in formulating the copolymer:

a) should be multifunctional, i.e., should have at least two reactive, polymerizable, unsaturated functional groups,
b) should contain a suitable combination of two or more unsaturated functional groups such as vinyl, allyl, acrylate, methacrylate in the same molecule.

Preferred chain branching monomers are those containing a combination of fast and slow reacting unsaturated groups. The fast reacting group is preferentially incorporated in the polymer backbone during the first step, whereas the slow reacting group reacts preferably in the second step.
According to the invention allyl methacrylate is used as the polyfunctional monomer.
The reactivity of one of the functional groups should be relatively lower than the reactivity of the other. Table 1 below shows the reactivity ratios, r1 and r2, for allyl, acrylic and methacrylic functional groups; as defined in the Polymer Handbook, by H. Immergut and J. Brandrup, 3rd Edition, Interscience, 1989. It can be seen that the allyl groups react 3 to 10 times slower than the other groups.

**Table 1 Reactivities of Functional Groups**

| Fast Monomer/Slow Monomer | r1 | r2 |
|---|---|---|
| Acrylic Acid/Allyl Acetate | 0.500 | 0.061 |
| Methacrylic Acid/Allyl Acetate | 1.129 | 0.066 |
| Ethyl Acrylate/Allyl Acetate | 0.600 | 0.165 |
| Methyl Methacrylate/Allyl Acetate | 0.383 | 0.136 |
| n-Butyl acrylate/Allyl Acetate | 0.427 | 0.199 |

The monomers B are hydrophilic, or water-soluble monomers which are sufficiently water soluble to form at least a ten-weight percent solution when dissolved in water and readily undergo additional polymerization to form polymers which are water soluble. The monomers B are acrylic acid, methacrylic acid or mixtures thereof.
Since monomers B are acidic, they make it possible for the resultant copolymer to be neutralized by reaction with an appropriate base so that the copolymer may exhibit a desirable level of water solubility. For example, the copolymer may be neutralized prior to being incorporated into an ultimate hair styling composition, allowing the composition to be removed from the hair simply by washing with water. Alternatively, if the copolymers are not pro-neutralized in this manner, removal may still be readily effected by application of an aqueous alkaline solution, such as soap in water.
The exact ratio of the monomers A and B is not critical to solubility. Copolymers with a high proportion of the hydrophobic A-Block can be dissolved in water by adjusting the pH.
In order to modify or enhance selected properties of the copolymer, for example, resistance to humidity, washability, and the like, the monomers A and B may be single monomers, or a combination of two or more monomers.
As for the actual preparation of the copolymer, any of the usual acrylate polymerization methods known in the art, such as solvent, suspension, emulsion, and inverse emulsion polymerization methods may be employed. In one preferred method of preparation of the copolymer, the monomers A, B, and X are reacted together in a suitable solvent. A free radical initiator is added in small quantities.
Suitable free radical initiators include azo- and peroxo-type initiators. Examples of azo-initiators are azobis-dimethylvaleronitrile, azobis-isobutyronitrile, azobis-methylbutyronitrile and others sold by DuPont, Wilmington, DE under the trade name VAZO and by WAKO Pure Chemical Industries, Richmond, VA under the trade name of V-40 to V501. Examples of peroxo initiators include di-t butyl peroxide, t-butyl cumyl peroxide, T-butyl peroxypivalate, lauryl peroxide, cumene hydroperoxide, ethyl hexyl peroxodicarbonate, diisopropyl peroxydicarbonate, 4-(t-butylperoxylperoxycarbonyl)-3-hexyl-6-7-(t-butylperoxycarbonyl)heptyl cyclohexene (4-TBPCH), cumene hydroperoxide and t-butyl peroxyneodecanoate, t-butyl hydroperoxide, benzoyl peroxide and other organic peroxides sold by Elf Atochem North America, Inc., Philadelphia, PA, under the trade names of Lupersol. Luperco, Lucidol and Luperox.
The initiator is preferably added at 0.005 mole percent to 1 mole percent of the total monomer composition. Preferred initiators are di-T-butyl peroxide, T-butyl cymyl peroxide, T-butyl peroxypivalate, lauryl peroxide, cumene hydroperoxide, ethyl hexyl peroxodicarbonate, diisopropyl peroxydicarbonate, 4-(t-butylperoxylperoxycarbonyl)-3-hexyl-6-7-(t-butylperoxycarbonyl) heptyl cyclohexene, cumene hydroperoxide and t-butyl peroxyneodecanoate, t-butyl hydroperoxide, benzoyl, peroxide and combinations thereof.
The polymerization can be carried in an variety of solvents, such alcohols, ethers, esters, aromatic solvents, glycols, glycol ethers, and glycol esters. Preferred solvents include ethyl alcohol, isopropyl alcohol, t-butyl alcohol, ethyl

acetate, methyl acetate, butyl acetate, benzene, toluene, and methylene choride. These solvents can be used also in combination with hydrocarbon solvents such as hexane, cyclohexane, mineral spirits, and the like. One preferred solvent is an isopropyl alcohol and water mixture.

Preferably, a reaction vessel, containing the solvent is heated to a suitable polymerization temperature. The monomers A; B, and X are metered, as a mixture, into the reaction vessel, over a period of several hours. Optionally, the mixture of monomers is varied throughout the reaction period. The initiator, dissolved in an additional portion of the solvent, is simultaneously metered into the reaction vessel.

The resulting copolymer can be dried and ground into a powder, or used directly from solution.

The weight of each of the monomers in the mixture can vary, depending on the desired properties of the copolymer. In one preferred embodiment, the monomer A for A-Block comprises from 28 to 60% by weight of the mixture of monomers, the chain extender monomer X comprises from 1 to 1.5 by weight of the mixture, and the monomer B for B-Block comprises from 38 to 69% by weight of the mixture.

The copolymers are suitable additives for the formulation of hair fixative formulations, such as aerosol and non-aerosol hair spray, spritz, gel, spray gel, mousse, styling creams, hair relaxers, and the like. The copolymer is compatible with dyes and pigments suitable to prepare colored hair fixatives. Since the copolymers are soluble in water and alcohol mixtures, they are suitable for the formulation of reduced volatile organic compounds (VOC) fixative formulations. The copolymers can be used to prepare 80%, 55%, 30%, or less VOC, and alcohol free formulations.

The copolymer is also suitable for the preparation of shampoos, conditioners, rinses, liquid soap, soap bars, detergents, cleaners, room deodorizers, and the like.

The copolymers are also suitable additives for the formulation of hair and skin creams, lotions, pomades, and ointments; topical medicated creams, skin protective films, hair depilatories, hair shaving creams, hand and body lotions, mascaras, sunscreens, and such.

The copolymer also finds application as additive in nail care formulations such as water-based nail polish, nail repair, nail protection, and the like because it is a film forming polymer which is removable due to the polymer having both hydrophobic and hydrophilic group.

The copolymer can also be used advantageously in the formulation of pharmaceutical formulations such as creams, pomades, gels, tooth paste, tablets, gel capsules, enema fluids, vomitives, suppositories, foamed anti-fungal preparations, drug delivery compositions to deliver transdermally active ingredients to or through the skin, ocular fluids, anti-acne formulations, topical analgesics, and the like.

The block/branched copolymers can be used in a host of applications where the presence of polymeric blocks with different properties is a useful property. They can be used as additives in cosmetic applications, body implants, coatings for catheters, cannulae, antiperspirant and deodorant formulations, coating for medical devices, gloves, removable protective coatings, wound dressings, etc. They can be used in the formulation of inks, protective washable coatings for textiles, fabrics, metal strippers, and the like.

In particular, the polymers of this invention are designed to provide a combination of long lasting hair style retention at high humidity, natural feel, good hair combing, reduced flaking, no build up, and good hair stylability and restyling. They are good film formers, washable with water and shampoo.

Formulations incorporating the copolymers may be delivered from aqueous or hydro-alcoholic solutions, dispersions, or emulsions. The copolymers can be dissolved in water, water-ethanol or water-solvent mixtures by dispersing the copolymer in the solvent and adjusting the pH with an organic or inorganic base between pH3 and pH12. A preferred pH is 5.0 to 9.0. Within this pH range, water clear solutions of the copolymer can be prepared.

In preparing hair styling compositions which incorporate the copolymer, the copolymer, either in powdered or liquid form, is combined with a solvent system, or with a solvent/propellant system. Preferably, the copolymer comprises between 0.01-20% by weight of the total weight of the composition, more preferably between 0.5-10% by weight. The solvent system preferably includes water and an organic solvent. Suitable organic solvents include alcohols, glycols and ketones, such as ethanol, isopropanol, acetone, dioxymethane, or methyl ethyl ketone, propylene glycol, hexylene glycol, and butylene glycol. For low VOC compositions, the solvent system preferably includes at least 20-50 weight percent water, and optionally up to 100% water. Preferably not more than about 25 weight percent of the organic solvent is used.

The hair styling compositions may be in the form of an aerosol or non-aerosol spray, a mousse, gel, or hair setting lotion. The compositions may contain up to 60 weight percent, preferably up to 35 weight percent, of liquified gases. Typical propellants include ethers, compressed gases, halogenated hydrocarbons and hydrocarbons. Exemplary propellants are dimethyl ether, compressed nitrogen, air or carbon dioxide, propane, butane, and 1,1 difluoroethane. Optionally, the solvent acts as the propellant.

The compositions may further include other materials or formulation additives, such as fragrances, preservatives, dyes and other colorants, plasticizers, emulsifiers, conditioners, neutralizers, glossifiers, lubricants, penetrants, UV absorbers, and the like. Mousses, according to the present invention, may further comprise from 0.25 to 6 weight percent, preferably 0.25 to 3 weight percent, of an emulsifier. The emulsifier may be nonionic, cationic, anionic, or amphoteric.

## Formulation Additives

Examples of additives are used in the formulation of hair, skin and nail products, include the following:

**Conditioning Agents:** In accordance with one important embodiment of the present invention, the composition of the present invention also includes from about 0.1% to 10%, particularly 0.5% to 10%, and preferably from 1.0% to 5.0%, by weight of a non-volatile silicone compound or other conditioning agent(s), preferably a water-insoluble, emulsifiable conditioning agent. The preferred non-volatile silicone compound is a polydimethylsiloxane compound, such as a mixture, in a 3:1 weight ratio, of a low molecular weight polydimethylsiloxane fluid and a higher molecular weight polydimethylsiloxane gum. The non-volatile polydimethylsiloxane compound is added to the composition of the present invention in an amount sufficient to provide improved combing and improved feel (softness) to the hair. As referred to herein, "silicone gums" are those nonfunctional siloxanes having a viscosity of from $5 \cdot 10^{-6}$ to $6 \cdot 10^{-1}$ $m^2/s$ (5 to 600,000) centistokes at 25 °C.

The so-called rigid silicones, as described in U.S.-A-4,902,499 having a viscosity above $0.6 m^2/s$ (600,000 centistokes) at 20 °C., e.g. 0.7 $m^2/s$ (700,000 centistokes) plus, and a weight average molecular weight of at least

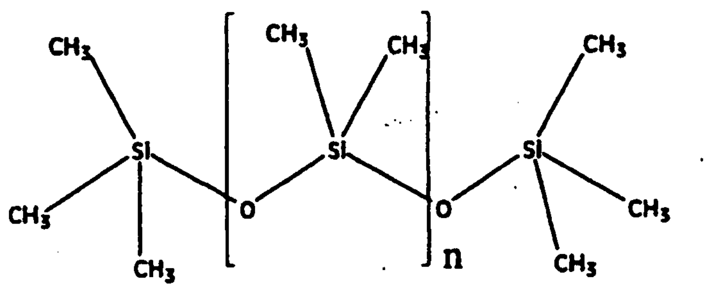

500,000 also are useful in accordance with the present invention. Preferred silicone gums include linear and branched polydimethylsiloxanes. Silicone gums useful in compositions of the present invention are available from a variety of commercial sources, including General Electric Company, Dow Coming and B.F.Goodrich.

Another particularly suitable conditioning agent that can be included in the composition of the present invention is a volatile hydrocarbon, such as a hydrocarbon including from 10 to 30 carbon atoms, that has sufficient volatility to slowly volatilize from the hair after application of the aerosol or non-aerosol styling aid composition. The volatile hydrocarbons provide essentially the same benefits as the silicone conditioning agents. The preferred volatile hydrocarbon compound is an aliphatic hydrocarbon including from 12 to 24 carbon atoms, and having a boiling point in the range of from 100°C to 300°C. Examples of volatile hydrocarbons useful in the composition of the present invention are the commercially-available compounds PERMETHYL 99A and PERMETHYL 101A, available from Permethyl Corporation, Frazer, Pennsylvania. A volatile hydrocarbon compound is useful in the composition of the present invention either alone, in combination with another volatile hydrocarbon, or in combination with a volatile silicone. Examples of other suitable water-insoluble conditioning agents that can be incorporated into the aerosol or non-aerosol aqueous styling aid composition of the present invention include the following: polysiloxane polyether copolymers; polysiloxane polydimethyl dimethylammonium acetate copolymers; acetylated lanolin alcohols; dimethyl dialkyl ammonium chlorides; modified alkyl dimethyl benzyl ammonium chlorides; lauryl dimethylamine oxide; stearyl dimethyl benzyl ammonium chloride; a lanolin-derived extract of sterol on sterol esters; lanolin alcohol concentrate; an isopropyl ester of lanolin fatty acids; sulfur rich amino acid concentrates; isopropyl ester of lanolin fatty acids; stearyl dimethyl benzyl ammonium chloride; cetyl trimethyl ammonium chloride; oleyl dimethyl benzyl ammonium chloride; oleyl alcohol; stearyl alcohol; stearyl dimethyl benzyl ammonium chloride; stearamidopropyl dimethyl myristyl acetate; a polyol fatty acid; a fatty amido amine; guar hydroxypropyltrimonium chloride; cetyl/stearyl alcohol; quaternized protein; keratin protein derivatives; isostearamidopropyl dimethylamine; stearamidopropyl dimethylamine; cetrimonium bromide; myrtrimonium bromide; stearalkonium chloride; cetyl trimethyl ammonium chloride; laurylpyridinium chloride; tris(oligoxyethyl)alkyl ammonium phosphate: an aminofunctional silicone; lapyrium chloride; isopropyl ester of lanolic acids; ethoxylated (30) castor oil; acetylated lanolin alcohol; fatty alcohol fraction of lanolin; a mineral oil and lanolin alcohol mixture; high molecular weight esters of lanolin; quaternium-75; vinylpyrrolidone/dimethylaminoethylmethacrylate copolymer; alkyl trimethyl ammonium chloride; 5 mole ethylene oxide adduct of soya sterol; 10 mole ethylene oxide adduct of soya sterol: stearic acid ester of ethoxylated (20 mole) methyl glucoside; sodium salt of poly-hydroxycarboxylic acid; hydroxylated lanolin; cocamidopropyl dimethylamine lactate; cocamidopropyl dimethylamine propionate; cocamidopropyl morpholine lactate; isostearamidopropyl dimethylamine lactate; isostearamidopropyl morpholine lactate; oleamidopropyl dimethylamine lactate; linoleamidopropyl dimethylamine lactate; stearamidopropyl dimethylamine lactate, ethylene glycol monostearate and propylene glycol mixture; stearamidopropyl dimethylamine lactate; acetamide MEA; lactamide MEA; stearamide MEA; behenalkonium

chloride; behenyl trimethyl ammonium methosulfate and cetearyl alcohol mixture; cetearyl alcohol; isostearamidopropalkonium chloride; linoleamidopropalkonium chloride; oleyl dimethyl benzyl ammonium chloride; tallow imidazolinum methosulfate; stearyl dimethyl benzyl ammonium chloride; stearyl trimonium methosulfate; mixed ethoxylated and propoxylated long chain alcohols; stearamidopropyl dimethylamine lactate; polonitomine oxide; oleamine oxide; stearamine oxide; soya ethyldimonium ethosulfate; hydroxypropyl bislauryl-dimonium chloride; hydroxypropyl biscetyl-dimonium chloride; hydroxypropyl bisstearyl dimonium chloride; hydroxypropyl bisbehenyl dimonium chloride; ricinolamidopropyl ethyldimonium ethosulfate; olealkonium chloride; stearalkonium chloride; N-(3-isostearamidopropyl)-N,N-dimethyl amino glycolate; N-(3-isostearamidopropyl)-N,N dimethyl amino gluconate; hydrolyzed animal keratin; ethyl hydrolyzed animal keratin; stearyl ammonium chloride; stearamidoethyl diethylamine; cocamidopropyl dimethylamine; lauramidopropyl dimethylamine; oleamidopropyl dimethylamine; palmitamidopropyl dimethylamine; stearamidopropyl dimethylamine lactate; avocado oil; sweet almond oil, grape seed oil; jojoba oil; apricot kernel oil; sesame oil; hybrid safflower oil: wheat germ oil; cocamidoamine lactate; ricinoleamido amine lactate; stearamido amine lactate; stearamido morpholine lactate; isostearamido amine lactate; isostearamido morpholine lactate; wheat germamido dimethylamine lactate; behenamidopropyl betaine; ricinoleamidopropyl betaine; wheat germamidopropyl dimethylamine oxide: disodium isostearaimido MEA sulfosuccinate; disodium oleamide PEG-2 sulfosuccinate: disodium oleamide MEA sulfosuccinate; disodium ricinoleyl MEA sulfosuccinate; disodium wheat germamido MEA sulfosuccinate; disodium wheat germamido PEG-2 sulfosuccinate; stearalkonium chloride; stearly dimethyl benzyl ammonium chloride; stearamido amine; stearamido morpholine; isostearamido amine: isostearamido morpholine; polyethylene glycol (400) mono and distearates; synthetic calcium silicate; isostearic alkanolamide; ethyl esters of hydrolyzed animal protein; blend of cetyl and stearyl alcohols with ethoxylated cetyl or stearyl alcohols; amido amines; polyamido amines: palmityl amido betaine; propoxylated (1-20 moles) lanolin alcohols; isostearamide DEA; and hydrolyzed collagen protein. When one or more of these water-insoluble conditioning agents is included in the composition of the present invention in an amount of 0.5% to 10% by total weight of the composition, the composition also can include a suspending agent for the conditioning agent, in an amount of 0.5% to 10%, by total weight of the composition. The particular suspending agent is not critical and can be selected from any materials known to suspend water-insoluble liquids in water. Suitable suspending agents are for example, distearyl phthalamic acid; fatty acid alkanolamides; esters of polyols and sugars; polyethylene glycols; the ethoxylated or propoxylated alkylphenols; ethoxylated or propoxylated fatty alcohols; and the condensation products of ethylene oxide with long chain amides. These suspending agents, as well as numerous others not cited herein, are well known in the art and are fully described in the literature, such as McCutcheon's Detergents and Emulsifiers, 1989 Annual, published by McCutcheon Division, MC Publishing Co. A nonionic alkanolamide also is optionally included in an amount of 0.1% to 5% by weight in the styling aid compositions that include a conditioning agent to provide exceptionally stable emulsification of water-insoluble conditioning agents and to aid in thickening and foam stability. Other useful suspending and thickening agents can be used instead of the alkanolamides such as sodium alginate; guar gum; xanthan gum; gum arabic; cellulose derivatives, such as methylcellulose, hydroxybutylcellulose, hydroxyethylcellulose, hydroxypropylcellulose and carboxymethylcellulose; and various synthetic polymeric thickeners, such as the polyacrylic acid derivatives. Suitable alkanolamides include, but are not limited to, those known in the art of hair care formulations, such as cocamide monoethanolamide (MEA), cocamide diethanolamide (DEA), soyamide DEA, lauramide DEA, oleamide monoisopropylamide (MIPA), stearamide MEA, myristamide MEA, lauramide MEA, capramide DEA, ricinoleamide DEA, myristamide DEA, stearamide DEA, oleylamide DEA, tallowamide DEA, lauramide MIPA, tallowamide MEA, isostearamide DEA, isostearamide MEA and combinations thereof.

**Neutralizing Agents:** In certain applications such as hair and skin care compositions, it is necessary to neutralize the hydrophilic B-block of the copolymer to achieve solubility or dispersibility. Neutralization and increased solubilization are accomplished, but not limited to, with one or more inorganic bases such as sodium hydroxide, potassium hydroxide, ammonium hydroxide and/or ammonium carbonate. Among stable organic bases are the water soluble alkanol amines such as monoethanolamine (MEA), diethanolamine (DEA), triethanolamine (TEA), 2-methyl-2-amino-1-propanol (AMP), monoamino glycols, and the like, which help solubilize the polymer in water solutions. The level of neutralization required for solubilization varies for each polymer. The block copolymers become soluble in water and hydroalcoholic solutions at 20% to 100% neutralization, and at all described levels of water/alcohol/propellant solutions. The pH of these solutions usually ranges from 4 to 12. The lowest neutralization level needed to render the polymer water soluble or dispersible depends on the composition of the block polymer, and the amount of alcohol, water, and propellant.

**Aerosol Propellant Gas:** The propellant gas included in the aerosol compositions of the present invention can be any liquefiable gas conventionally used for aerosol containers. Examples of materials that are suitable for use as propellants are trichlorofluoromethane, dichlorodifluoromethane, dichlorotetrafluoroethane, monochlorodifluoromethane, trichlorotrifluoroethane, dimethyl ether, propane, n-butane and isobutane, used singly or admixed. Water-soluble gases such as dimethyl ether, carbon dioxide, and/or nitrous oxide also can be used to obtain aerosols having reduced flammability. Water-immiscible, liquified, hydrocarbon and halogenated hydrocarbon gases such as propane, butane and chlorofluorocarbons can be used advantageously to deliver the contents of the aerosol container without the dramatic pressure drops associated with other immiscible gases. Here there is no concern for the head space to be left inside

the aerosol container, because the liquified gas will sit on top of the aqueous formulation and the pressure inside the container is always the vapor pressure of saturated hydrocarbon vapor. Other insoluble, compressed gases such as nitrogen, helium and fully-flourinated oxetanes and oxepanes also are useful to deliver the compositions from aerosol containers. Other means of delivery of the above-described aqueous styling aid compositions include, pump sprayers, all forms of bag-in-can devices, in situ carbon dioxide ($CO_2$) generator systems, compressors, and the like. The amount of the propellant gas is governed by normal factors well known in the aerosol art. For mousses, the level of propellant is generally from 3% to 30%, preferably from 5% to 15% of the total composition. If a propellant such as dimethyl ether utilizes a vapor pressure suppressant (e.g., trichlorethane or dichloromethane), for weight percentage calculations, the amount of suppressant is included as part of the propellant.

**Optional Additives:** The hair styling compositions also can contain a variety of other nonessential, optional components suitable for rendering such compositions more aesthetically acceptable. Such conventional optional ingredients are well known to those skilled in the art, e.g., other emulsifiers such as anionics (e.g., sodium alkyl sulfate); preservatives such as benzyl alcohol, methyl paraben, propyl paraben and imidazolidinylurea; cationic emulsifiers/conditioners such as cetyl trimethyl ammonium chloride, stearyldimethyl benzyl ammonium chloride, and di(partially-hydrogenated tallow) dimethylammonium chloride; viscosity modifiers such as a diethanolamide of a long chain fatty acid, fatty alcohols (i.e., cetearyl alcohol), sodium chloride, sodium sulfate, and ethyl alcohol; pH adjusting agents such as citric acid, succinic acid, sodium hydroxide and triethanolamine; coloring agents such as any of the FD&C or D&C dyes; hair oxidizing (bleaching) agents such as hydrogen peroxide, perborate salts and persulfate salts; hair reducing agents such as thioglycolates; perfume oils; chelating agents such as ethylenediaminetetraacetic acid; and, among many other agents, polymer plasticizing agents such as glycerin and propylene glycol. These optional materials are generally used individually at a level of from 0.01% to 19%, preferably from 0.5% to 5% by weight of the total composition. The aqueous formulations of the present invention also can contain the conventional hair spray adjuvants in amounts which generally range from 0.1 to 2% by weight and preferably 0.75 to 1% by weight. Among the additives which can be used are plasticizers such as glycols, phthalate esters and glycerine; silicones; emollients; lubricants and penetrants such as various lanolin compounds; protein hydrolysates and other protein derivatives; ethylene adducts and polyoxyethylene cholesterol; dyes, tints and other colorants; and perfumes.

**Carrier Vehicle**: Polar solvents are typically used to prepared the cosmetic or hair compositions. Water, glycols and alcohols are preferably used. The optional alcohol employed in the composition is an aliphatic straight or branched chain monohydric alcohol having 2 to 4 carbon atoms. Isopropanol and especially ethanol are preferred. The concentration of the alcohol in the composition should be less than 40% by weight, and surprisingly can be as low as 0%, preferably 0-30% by weight and more preferably 5-20% by weight. Some alcohol, in an amount of 2% to 10% by weight.

In preparing the hair styling compositions of the present invention, the copolymer is optionally neutralized to the extent that from about 40 to 100 mole percent of the carboxylic groups in the copolymer are neutralized by a neutralization agent, in a solvent system wherein water is the primary solvent. At neutralizations greater than around 92%, the humidity resistance of the hair styling composition is greatly reduced.

The hair styling compositions incorporating the branched block copolymer exhibit desirable characteristics of such compositions, including long lasting hair style retention at high humidity, natural feel, good hair combing, reduced tack, reduced flaking, good stylability and restyling, no fly away, and the like.

Other applications for the polymers of the present invention would include: adhesive, metal cleaners, oil/gas well cleaners, preservative intermediate, polystyrene manufacture, surface cleaners, industrial cleaners, coatings, industrial, coatings, specialty and imaging, paint stripper, printing inks, photo-resist applications, paints/latex systems, battery manufacture, reaction solvent and fiber dying, detergents, textile dye stripping, printed circuit boards, dispersants, gel former, petrochemical processing, and paper manufacture.

While not intended to limit the invention, the following examples are illustrative of the methods of preparing the copolymers and hair styling compositions, and of their unique styling properties.

**General Method Or Preparation Of Polymers**

The following method was used to prepare a variety of copolymers. A clean, dry 8 liter glass reactor was set up in a heated waterbath and fitted with a condenser and a stirring agitator. To the reactor, 1750 grams of solvent, a mixture of 80% isopropyl alcohol and 20% water, was charged and heated to reflux temperature (78-82°C). A first batch, or feed of monomers for forming the A-Block, 1200 grams of n-butyl acrylate, and 116 grams of methacrylic acid, and 20 grams of a chain extender, allyl methacrylate, were mixed together and charged into a storage cylinder connected to the reactor by a feed line and a metering pump. 16.8 grams of an initiator, t-butyl peroxypivalate were diluted with a further 200 grams of solvent and charged to another storage cylinder also connected to the reactor by a metering pump.

After the reactor reached reflux temperature the polymerization was started. The first batch of monomers was fed in evenly over a one hour period and the initiator was fed in over evenly over a four hour period. After the first hour, a second batch, or feed, of monomers for forming the **B-Block**, a blend of 1053 grams of methacrylic acid and 10 grams

of allyl methacrylate chain extender was fed into the reactor evenly over a two hour period. The total monomer feed time was three hours and the total initiator feed time was four hours. After all the ingredients had been added, the reactor was held at reflux temperature for another two hours before cooling to room temperature.

The resulting reaction mixture comprised 50 percent solids by weight.

The above method was used to prepare copolymers of varying compositions. The molecular weight (Mw) of the polymers was measured by gel permeation chromatography, GPC. The $T_g$ of the polymers was measured using differential scanning calorimetry, DSC. Table 2 summarizes the quantities of the three monomers used in preparing each of the copolymers, in terms of the weight added in each of the two feed mixtures, and the total weight of monomer added in both the mixtures. The percentage solids in the reactor at the end of the reaction period is included for each of the copolymers prepared, together with the high and low glass transition temperatures of the copolymer, and the molecular weight of the copolymer.

**Key for the Examples of Polymers:**

In the examples, the following abbreviations will be used:

| | | |
|---|---|---|
| nBA | = | n-butyl acrylate |
| All MA | = | (chain extender) allyl methacrylate |
| AA | = | acrylic acid |
| MAA | = | methacrylic acid |
| EGDMA | = | (chain extender) ethylene glycol dimethacrylate |
| Lup-11 | = | (initiator) t-butyl peroxypivalate (Lup-11 is short for Lupersol-11, and is available from Atochem North America, Inc.) |
| IPA/$H_2O$ | = | (solvent) at 80/20 wt % mixture of isopropyl alcohol/water |
| Tg 1 | = | Low glass transition temperature |
| Tg 2 | = | High glass transition temperature |
| phm | = | parts per hundred parts of monomer |
| Mw | = | weight average molecular weight |

**Substantiation Examples of Block/Branched Copolymers with Two Tg**

The copolymers in Examples 1 to 3 were prepared following the polymerization scheme above. The A-block was prepared using two monomers and the B-block contains only one. The A-Block is predominantly hydrophobic; the B-block is hydrophilic and ionizable. The molecular weight Mw of the polymers was measured by gel permeation chromatography, GPC. The Tg of the polymers was measured using differential scanning calorimetry, DSC.

**TABLE 2**

| **Example** | **Monomer Composition** | | | | | ***Observations*** |
|---|---|---|---|---|---|---|
| | | ***A-Block*** | ***B-Block*** | ***Total Batch*** | | |
| | ***Ingredient*** | ***(g)*** | ***(g)*** | ***(g)*** | ***phm*** | |
| **1** | AA | 216.00 | 1944.00 | 2160.00 | 60.00 | |
| | n-BA | 1386.00 | 0.00 | 1386.00 | 38.50 | *Mw = 88,900* |
| | All MA | 36.00 | 18.00 | 54.00 | 1.50 | *Tg 1 = -13.7°C* |
| | Lup - 11 | 18.00 | | 18.00 | 0.50 | *Tg 2 = 97°C* |
| | IPA/H2O | | | 2400.00 | 66.66 | |
| **2** | AA | 162.40 | 1461.60 | 1624.00 | 70.00 | |
| | n-BA | 661.20 | 0.00 | 661.20 | 28.50 | *Mw = 67,500* |
| | All MA | 34.80 | 18.00 | 52.80 | 1.50 | *Tg 1 = 19°C* |
| | Lup - 11 | 16.24 | | 16.24 | 0.50 | *Tg 2 = 106°C* |
| | IPA/H2O | | | 2400.00 | 66.66 | |

(continued)

| Example | Monomer Composition | | | | | Observations |
|---|---|---|---|---|---|---|
| | | *A-Block* | *B-Block* | *Total Batch* | | |
| | *Ingredient* | *(g)* | *(g)* | *(g)* | *phm* | |
| **3** | MAA | 175.00 | 1580.00 | 1755.00 | 48.75 | |
| | n-BA | 1800.00 | | 1800.00 | 50.00 | *Mw = 59.200* |
| | All MA | 30.00 | 15.00 | 45.00 | 1.25 | *Tg 1 = -17°C* |
| | Lup- 11 | | | 25.2 | 0.70 | *Tg 2 = 145°C* |
| | IPA/H2O | | | 2400.00 | 66.66 | |

**Examples to Substantiate the Criticality of Using a Suitable Chain Extender**

To demonstrate the blocky-structure of the polymer and the importance of using a suitable chain extender, block copolymers were prepared where the A-block was a hydrophobic, water-insoluble block and the B-block, hydrophilic and ionizable. Examples 4 and 5 were prepared in a similar manner, without and with allyl methacrylate, All MA (the chain extender monomer), respectively. The hydrophobic block of Polymer 4, after neutralizing with base at high pH, did not dissolve in water. The polymer formed a milky, phase-separated suspension. Conversely, the polymer of Example 5, which includes All MA, formed a water clear solution after neutralization with base at pH = 7.16.

**TABLE 3**

| Example | Monomer Composition | | | | |
|---|---|---|---|---|---|
| | | *A-Block* | *B-Block* | *Total Batch* | |
| | *Ingredient* | *(g)* | *(g)* | *(g)* | *phm* |
| | | | | | |
| **4** | MAA | 446.00 | 1354 | 1800.00 | 60.00 |
| | n-BA | 1200.00 | 0.00 | 1200.00 | 40.00 |
| | All MA | 0 | 0 | 0 | 0 |
| | Lup - 11 | | | 21.00 | 0.70 |
| | IPA/H2O | | | 3000.00 | 100.00 |
| **5** | MAA | 446.00 | 1316.5 | 1762.5 | 58.75 |
| | n-BA | 1200.00 | 0.00 | 1200.00 | 40.00 |
| | All MA | 25.0 | 12.5 | 37.5 | 1.25 |
| | Lup- 11 | | | 21.00 | 0.70 |
| | IP A/H2O | | | 3000.00 | 100.00 |
| **6*** | MAA | 446.00 | 1316.5 | 1762.50 | 58.75 |
| | n-BA | 1200.00 | 0.00 | 1200.00 | 40.00 |
| | EGDMA | 25.00 | 5.0 | 30.00 | 1.00 |
| | Lup - 11 | | | 30.00 | 1.00 |
| | IPA/H2O | | | 3000.00 | 100.00 |
| *) not according to the invention | | | | | |

Resin of Example 6 (not according to the invention) was prepared using ethylene glycol dimethacrylate, EGDMA, a difunctional chain extender whose reactive groups have comparable reactivity. The resulting polymer was heavily cross-linked and formed a solid gelled mass during polymerization. The polymer was impossible to isolate and test. In

contrast the polymer of Example 5, prepared with allyl methacrylate, formed an easy-to-handle viscous liquid, during polymerization.

As can be seen from Table 2, varying the composition of monomers allows for the preparation of copolymers of different molecular weights and glass transition temperatures, which permits modification of the desirable properties of the hair styling compositions formulated with the copolymers.

## Examples of Block Copolymers for Hair Styling Aid Formulations

A hair fixative resin should also encompass a number of subjective and objective properties such as curl ease of formulation, sprayability, feel on the hair, washability, curl retention, fast drying and low tack, compatibility with ancillary formulation additives, etc.

The following examples show that blocky/branched copolymers were prepared to demonstrate that, hair fixative polymers with superior performing properties can be obtained by varying the hydrophilic and hydrophobic character of the A and B blocks.

TABLE 4

| **Example** | **Monomer Composition** | | | | | ***Observations*** |
|---|---|---|---|---|---|---|
| | | ***A-block*** | ***B-block*** | ***Total Batch*** | | |
| | ***Monomer*** | ***(g)*** | ***(g)*** | ***(g)*** | ***phm*** | |
| **7** | MAA | 531.00 | 511.50 | 1042.50 | 34.75 | *Forms clear* |
| | AA | 144.00 | 576.00 | 720.00 | 24.00 | *Solution* |
| | n-BA | 1200.00 | | 1200.00 | 40.00 | *At pH = 9.93* |
| | All MA | 25.00 | 12.50 | 37.50 | 1.25 | |
| | Lup - 11 | 52.50 | 0.00 | 52.50 | 1.75 | |
| | IPA/H2O | | | 3000.00 | 100.00 | |
| **8** | MAA | 531.00 | 511.50 | 1042.50 | 34.75 | *Forms clear* |
| | AA | 720.00 | 0.00 | 720.00 | 24.00 | *Solution* |
| | n-BA | 1200.00 | | 1200.00 | 40.00 | *At pH = 5.44* |
| | All MA | 25.00 | 12.50 | 37.50 | 1.25 | |
| | Lup - 11 | 52.50 | 0.00 | 52.50 | 1.75 | |
| | IPA/H2O | | | 3000.00 | 100.00 | |
| **9** | MAA | 531.00 | 511.50 | 1042.50 | 34.75 | *Forms clear* |
| | AA | 576.00 | 144.00 | 720.00 | 24.00 | *Solution* |
| | n-BA | 1200.00 | | 1200.00 | 40.00 | *At pH = 8.2* |
| | All MA | 25.00 | 12.50 | 37.50 | 1.25 | |
| | Lup- 11 | 52.50 | 0.00 | 52.50 | 1.75 | |
| | IPA/H2O | | | 3000.00 | 100.00 | |
| **10** | MAA | 209.00 | 833.50 | 1042.50 | 48.75 | *Forms clear* |
| | AA | 720.00 | 0.00 | 720.00 | 10.00 | *Solution* |
| | n-BA | 1200.00 | | 1200.00 | 50.00 | *At pH = 5.5* |
| | All MA | 25.00 | 12.50 | 37.50 | 1.25 | |
| | Lup - 11 | 52.50 | 0.00 | 52.50 | 1.75 | |
| | IPA/H2O | | | 3000.00 | 100.00 | |

Note that by altering the hydrophilic hydrophobic balance of the A-block polymers soluble within a range of pH

were made. Polymers that dissolve at relatively lower pH such as the polymers from Examples 8 and 10 are desired for hair fixatives. Polymers that dissolve at higher pH would be more suitable for formulations where high pH is a benefit, i.e., depilatories, medicated creams, etc.

**Spravability:** Hair spray products are typically formulated in hydroalcoholic formulations. It is required that hair fixative resin produces a low viscosity formulations that can be aesthetically delivered in the form a fine spray. The data on Table 5 shows that the Block/Branched copolymers of examples 7, 8, and 9 have better sprayability than current art.

**Hair Feel:** The tactile feel that the hair acquires after been coated with a fixative resin is extremely important. Current polymers tend to leave the hair raspy, dry, gummy, grease, etc. The data in Table 5 shows that the copolymers 7, 8, and 9 have superior feel characteristics. They leave the hair soft and natural.

**Tack:** Most current fixative polymers tend to absorb moisture and therefore become tacky. Note that copolymers 7, 8, and 9 exhibit low tack.

**Flake-off:** Fixative polymers, after drying on hair, exhibit high levels of flakes after combing, giving the hair a dandruff-like appearance. Copolymers 7, 8, and 9 exhibit the lowest levels of flaking.

**TABLE 5**

(Subjective properties were evaluated directly on hair tresses. 1= worst, 10= best)

| Polymer | Hair Feel | Tack | Flake off | Sprayability | % Set Retention 1 hr, 90% RH |
|---|---|---|---|---|---|
| PVP, * | 3 | 2 | 2 | 1 | 30.00 |
| Amphomer ** | 2 | 3 | 8 | 2 | 80.00 |
| Lovocryl L73 *** | 2 | 3 | 8 | 2 | 50.00 |
| Luv VA73 | 4 | 6 | 6 | 3 | 30.00 |
| Luv Hold. | 4 | 6 | 6 | 3 | 30.00 |
| Example 7 | 6 | 5 | 8 | 6 | 30.00 |
| Example 8 | 6 | 5 | 8 | 8 | 90.00 |
| Example 9 | 5 | 5 | 8 | 7 | 84.00 |
| Example 10 | 5 | 5 | 3 | 3 | 100.00 |

* PVP is polyvinyl pyrrolidone
** Amphomer is a polymer sold by the M.H. Starch Co.
*** Lovocryl, Luv are trade names for polymers sold by BASF

An important performance property that a hair fixative polymer must also have, is its ability to hold a hairstyle in place at relatively high humidity, i.e., Curl Retention. The curl retention ability of the copolymers of this invention was measured and compared against a number of current hair fixative polymers.

**Curl Retention Protocol**: 0.05 grams of resin dissolved in a hydroalcoholic solution was applied and smeared on clean. 2 grams. 6 in, hair swatches. The swatches were rolled over salon rollers, dried and conditioned overnight. The swatches were mounted inside a humidity chamber at 80°F. and 90 % of relative humidity.

The curl retention was recorded as a function of time and calculated as:

$$L-L(t)/L-L(o)x100=\text{curl retention \%}$$

Where:

L=length of hair fully extended, L(o)=length of hair before exposure to high humidity,
L(t)=length of hair after exposure at time(t).

As shown in Table 5, the curl retention ability of the blocky-branched copolymers of Examples 8, 9, and 10 was superior to most current fixative polymers.

Low VOC hair styling compositions were prepared using the copolymers of Example 2. The compositions included 3-5 weight percent of a resin containing 60% weight percent of one of the copolymers of Table 2, a solvent

system, comprising ethanol and water, and a surfactant, AMP-95. All the compositions were formulated to 50% by weight VOC's. Table 6 lists the components of compositions and summarizes the subjective assessments. The compositions show improved performance over conventional, widely used hair styling formulations. The improved performance is seen in one or more of the following attributes: style retention at high humidity, natural feel, combability, reduced flaking, good styleability and restyleability.

**TABLE 6: Hair Styling Compositions Containing 50% VOC**

| | | |
|---|---|---|
| **Composition A** | | |
| Ingredient | % wt | Comments: |
| Polymer of Example 1<br>Ethanol<br>Amp 95*<br>Deionized water | 3.00<br>50.00<br>0.30<br>46.70 | Feel of hair is slick initially then it has a very touchable feel when completely dry. |
| **Composition B** | | |
| Ingredient | % wt | Comments: |
| Polymer of Example 2<br>Ethanol<br>Amp 95<br>Deionized water | 5.00<br>50.00<br>0.50<br>44.50 | Same as for composition A. |
| **Composition C** | | |
| Ingredient | %wt | Comments: |
| Polymer of Example 3<br>Ethanol<br>Amp 95<br>Deionized water | 3.00<br>50.00<br>0.17<br>46.83 | Feel of hair is slick initially then becomes touchable when dry. Humidity resistance is greater than for conventional PVP and PVP/vinyl acetate in alcohol formulations. |
| **Composition D** | | |
| Ingredient | % wt | Comments: |
| Polymer of Example 3<br>Ethanol<br>Amp 95<br>Deionized water | 5.00<br>50.00<br>0.29<br>44.71 | Same as for Composition C. |
| * AMP 95 is amino methyl propanol, 95% wt. in water | | |

Swatches of hair were sprayed or applied with the hair styling compositions in Table 6. The swatches were evaluated for humidity resistance, expressed in terms of percentage droop (in relation to a fully extended swatch of hair). Subjective assessments of natural feel. combability, resistance to flaking, and restylability/stylability were also made, on a 1 to 10 scale, 10 being the optimum. Table 8 summarizes these characteristics for the four hair styling compositions in Table 6.

**TABLE 7. Hair Styling Properties of Compositions Including the Copolymer**

| Composition | Humidity Resistance | Natural Feel | Combability | Flaking | Restylability Stylability |
|---|---|---|---|---|---|
| A | 30 min - 76% curl drop.<br>45 min - curl drop | 6 | 9 | 8 | 7 |
| B | 30 min - 92% curl drop.<br>1 hr. - curl drop | 6 | 9 | 7 | 7 |
| C | 7 hrs. - 88% curl drop,<br>24 hrs. - full droop | 6 | 9 | 10 | 8 |
| D | 46 hrs - 88% curl drop, | 6 | 9 | 8 | 8 |

(continued)

| Composition | Humidity Resistance | Natural Feel | Combability | Flaking | Restylability Stylability |
|---|---|---|---|---|---|
| | 72 hrs. - full droop | | | | |

As seen from Table 7, all of the compositions exhibited better than average natural, feel, combability, resistance to flaking, and stylability/ restylability characteristics. For hair styling compositions employing the same (composition A and B or C and D), these characteristics were rated equally for compositions having lower resin concentration (3%) and those with a higher resin concentration (5%), with the exception of resistance to flaking, which showed a marginal improvement at lower resin concentrations. Humidity resistance was greater at higher resin concentrations.

**Examples of Reduced VOC Aerosol Hair Fixative Formulations**

Example E. 55% VOC Aerosol Hair Spray Using Dimethyl Ether

| Item No. | Ingredient | Wt% |
|---|---|---|
| 1 | SD 40-200 Alcohol | 25.0 |
| 2 | Water | 35.0 |
| 3 | Example 5 | 8.0 |
| 4 | AMP-95 | 2.0 |
| 5 | Dimethyl Ether | 30.0 |

Items 1 thru 4 added and mixed in a container until a clear solution is obtained. This formulation was placed in an aerosol hair spray can. The can was capped with a standard aerosol actuator. Item 5 was pressure charged into the can. Upon discharging the product, the spray pattern was excellent, a very fine aerosol mist was obtained.

Example F. 55% VOC Aerosol Hair Spray Using 152A Propellant

| Item No. | Ingredient | Wt % |
|---|---|---|
| 1 | SD 40-200 Alcohol | 55.0 |
| 2 | Polymer of Example 5 | 8.0 |
| 3 | AMP-95 | 2.0 |
| 4 | Dymel 152A | 35.0 |

Items 1 thru 4 were added and mixed in a container until a clear solution is obtained. This formulation was placed in an aerosol hair spray can. The can was capped with a standard aerosol actuator. Item 5 was pressure charged into the can. Upon discharging the product, the spray pattern was excellent; a very fine aerosol mist was obtained.

Example G. Styling Mousse

| Item No. | Ingredient | Wt % |
|---|---|---|
| 1 | Water | 81.0 |
| 2 | Polymer of Example 5 | 3.5 |
| 3 | Emulphor on-870 | 0.5 |
| 4 | Propellant A-46 | 15.0 |

Item 1 thru 3 were added and mixed in a container until a clear solution is obtained. This formulation was placed in an aerosol mousse can. The can was capped with a standard mousse actuator. Item 4 was pressure charged into the can.

Upon discharging the product, a thick and creamy foam was obtained.

The following examples are intended to illustrate the range of uses for the film forming copolymers of this invention:

Ultrasonic Diagnosis Gel

0.5% Carbomer thickener
2.0% Polymer of Example 7
0.25% of NaOH
5.0% of glycerol to 100% with water + preservative

Ointment with Zinc Oxide

1.2% Polymer of Example 10
1.0% triethanolamine
14.0% of zinc oxide to 100% with water + preservative

Furniture Polish

1.0% Polymer of Example 10
5.0% silicone oil emulsions (30% strength)
3.0% carnauba wax emulsion (20% strength) to 100% with water

Domestic Cleaning Agent

1.5% Polymer of Example 10
1.3% triethanolamine
10.0% isopropyl alcohol
10.0% nonylphenol + 10 moles of ethylene oxide to 100% water

Water-In-Oil Cream

0.5% Polymer of Example 10
0.1% monoethanolamine
3.5% diglycerol sesquiissostearate
10.0% paraffin wax
5.0% cetyl alcohol
2.2% microwax
0.2% perfume oil to 100% water + perservative

After Shave Gel

1.1% Polymer of Example 10
0.4% monoethanolamine
35.0% ethyl alcohol
0.1 % menthol to 100% with water + preservative

Hair Shampoo

0.5% Polymer of Example 10
0.6% triethanolamine
12.0% coconut oil alcohol + 10 moles of ethylene oxide
0.1 % perfume oil to 100% of water + preservative

Hand Sanitizer

1.0% Polymer of Example 10
65.0% ethyl alcohol

1.5% carbopol
1.4% triethanolamine
0.1% perfume oil to 100% with water + preservative

Liquid Oil-in-Water Emulsion

0.5% Polymer of Example 10
0.2% NaOH
5.0% isopropyl palmitate
5.0% paraffin oil
5.1% diglycerol stearate + 4 moles of ethylene oxide
0.1% perfume oil to 100% with water + preservative

Oil-In-Water Cream

0.7% Polymer of Example 10
0.6% AMP-95
5.0% petrolatum
5.2% soybean oil
3.0% glycerol monostearate
3.0% tri-stearyl tetraglycol ether ortho-phosphoric acid to 100% with water + preservative

Liquid Water-In-Oil Emulsion

0.5% Polymer of Example 10
0.6% ammonium hydroxide (10% strength)
3.0% hydrogenated castor oil + 7 moles of ethylene oxide
2.0% polyglyceryl-2 sesquiisostearate
1.0% beeswax
1.0% mineral oil
0.5% magnesium stearate
0.5% aluminum montanate
10.0% isopropyl palmitate
15% perhydrosqualene to 100% with preservative + water

**Claims**

**1.** A film forming block copolymer formed in the presence of a free radical initiator from:

- a polyfunctional monomer having two functional groups, the reactivity of one functional group being higher than that of other functional group;
- first ethylenically unsaturated monomers which copolymerize preferentially with the functional group of the difunctional monomer which has a higher reactivity to form a first block; and
- a second ethylenically unsaturated monomer or monomers, which contains at least one carboxylic acid group and copolymerizes with the functional group of the difunctional monomer which has the lesser reactivity to form a second block, wherein the first block is more hydrophobic than the second block, and the copolymer has both hydrophobic and hydrophilic blocks, which have at least two different glass transition temperatures, wherein

the polyfunctional monomer is allyl methacrylate, the first ethylenically unsaturated monomers is a mixture of n-butyl acrylate and (meth)acrylic acid and the second ethylenically unsaturated monomer(s) is selected from the group consisting of acrylic acid, methacrylic acid and mixtures thereof.

**2.** The copolymer of claim 1, wherein:

the polyfunctional monomer comprises from 0.005 to 2 mole percent of the total monomers;
the first ethylenically unsaturated monomers comprise from 5 to 95 mole percent of the total monomers; and,
the second ethylenically unsaturated monomer(s) comprises from 5 to 70 mole percent of the total monomers.

3. The copolymer of claim 1, wherein:

the polyfunctional monomer comprises from 0.1 to 1.5 mole percent of the total monomers;
the first ethylenically unsaturated monomers comprise from 5 to 50 mole percent of the total monomers; and,
the second ethylenically unsaturated monomer(s) comprises from 10 to 70 mole percent of the monomers.

4. The copolymer of claim 1, wherein the copolymer has a molecular weight of less than 1, 000,000.

5. The copolymer of claim 1, wherein the first block has a molecular weight of 10,000 to 100,000.

6. The copolymer of claim 1, wherein the second block has a molecular weight of 1,000 to 100,000.

7. The copolymer of claim 1, wherein said first block has a glass transition temperature which is less than the glass transition temperature of said second block.

8. The copolymer of claim 1, wherein the first ethylenically unsaturated monomers is a mixture of n-butyl acrylate and methacrylic acid.

9. The copolymer of claim 8, wherein said acid monomer comprises 50 mole % or less of said monomer mixture.

10. The copolymer of claim 1, wherein the second ethylenically unsaturated monomers are a blend of acrylic acid and methacrylic acid.

11. The copolymer of claim 1, wherein the first ethylenically unsaturated monomers are a mixture of n-butyl acrylate and methacrylic acid, the second ethylenically unsaturated monomers are a blend of acrylic acid and methacrylic acid, and the acid monomers comprise 50 to 70 mole % of the total monomers.

12. The copolymer of claim 1, wherein said first block has a glass transition temperature of 30 °C or less and said second block has a glass transition temperature of greater than 30 °C.

13. The copolymer of claim 1, wherein said first block has a glass transition temperature of 0 °C or less and said second block has a glass transition temperature of greater than 0 °C.

14. The copolymer of claim 1, wherein the second block is neutralized.

15. A hair styling composition including from 0.01-20% by weight of the film forming branched block copolymer as defined in any of claims 1 to 14.

16. The hair styling composition of claim 15, further including at least one of the following materials:

0 to 25 weight percent of an emulsifier;
0.05 to 99% solvents;
0.05 to 10% rheology modifiers;
0.05 to 5% neutralizing agents;
0-60 weight percent of a liquid propellant or gas; and,
0-1% of a surfactant.

17. A method of preparing a hair styling composition, the method including preparing the film forming branched block copolymer as defined in any of claims 1 to 14.

18. The method of claim 17, wherein the preparation of the copolymer includes:

adding to a reaction vessel, a solvent, the polyfunctional monomer, the first ethylenically unsaturated monomers, and an initiator;
reacting the monomers to form a first block;
adding the second ethylenically unsaturated monomer or monomers; and,
reacting the monomers to form a second block and a copolymer having both hydrophobic and hydrophilic groups and at least two glass transition temperatures.

**19.** The method of claim 18, wherein the initiator is selected from the group consisting of azo-type initiators and peroxo-type initiators.

**20.** The method of claim 19, wherein the initiator is an azo-type initiator selected from the group consisting of azobis-dimethylvaleronitrile, azobis-isobutyronitrile, azobis-methylbutyronitrile, and combinations thereof.

**21.** The method of claim 19, wherein the initiator is a peroxo-type initiator selected from the group consisting of di-t-butyl peroxide, t-butyl cumyl peroxide, t-butyl peroxypivalate, lauryl peroxide, cumene hydroperoxide, ethyl hexyl peroxodicarbonate, diisopropyl peroxydicarbonate, 4-(t-butylperoxylperoxycarbonyl)-3-hexyl-6-7-(t-butylperoxycarbonyl)heptyl cyclohexene, cumene hydroperoxide and t-butyl peroxyneodecanoate, t-butyl hydroperoxide, benzoyl peroxide, and combinations thereof.

**22.** The method of claim 21, wherein the initiator is t-butyl peroxypivalate.

**23.** The method of claim 19, wherein the initiator is at a concentration of from 0.005 to 1 mole percent of the total monomers.

**24.** The method of claim 18, wherein the solvent is selected from the group consisting of water, hydrocarbons, alcohols, ethers, esters, aromatic solvents, glycols, glycol ethers, glycol esters, and combinations thereof.

**25.** The method of claim 24, wherein the solvent also includes water.

**26.** The method of claim 24, wherein the solvent is selected from the group consisting of water, ethyl alcohol, isopropyl alcohol, t-butyl alcohol, ethyl acetate, methyl acetate, butyl acetate, benzene, toluene, methylene chloride, hexane, cyclohexane, mineral spirits, and combinations thereof.

**27.** The method of claim 24, wherein the solvent is isopropyl alcohol and water.

**28.** The method of claim 18, wherein the copolymer is produced in the same reaction vessel.

**29.** The method of claim 18, further including neutralizing the copolymer so that between 0.1 and 100 percent of the carboxylic acid groups are neutralized.

**30.** Use of the film forming branched block copolymer as defined in any of claims 1 to 14

- as additives for the formulation of hair fixative formulations selected from aerosol and non-aerosol hair spray, spritz, gel, spray gel, mousse, styling creams, and hair relaxers;
- for the preparation of shampoos, conditioners, rinses, liquid soap, soap bars, detergents, cleaners, and room deodorizers;
- as additives for the formulation of hair and skin creams, lotions, pomades, and ointments; topical medicated creams, skin protective films, hair depilatories, hair shaving creams, hand and body lotions, mascaras, and sunscreens;
- as an additive in nail care formulations selected from water-based nail polish, nail repair, and nail protection;
- for the formulation of pharmaceutical formulations selected from creams, pomades, gels, tooth paste, tablets, gel capsules, enema fluids, vomitives, suppositories, foamed anti-fungal preparations, drug delivery compositions to deliver transdermally active ingredients to or through the skin, ocular fluids, anti-acne formulations, and topical analgesics;
- as additives in cosmetic applications, body implants, coatings for catheters, cannulae, antiperspirant and deodorant formulations, coating for medical devices, gloves, removable protective coatings, and wound dressings; and
- in the formulation of inks, protective washable coatings for textiles, fabrics, and metal strippers.

**Patentansprüche**

**1.** Filmbildendes Blockcopolymer, gebildet in Gegenwart eines Radikalstarters aus:

- einem polyfunktionellen Monomer mit zwei funktionellen Gruppen, wobei die Reaktivität der einen funktionellen

Gruppe höher ist als die der anderen funktionellen Gruppe;
- ersten ethylenisch ungesättigten Monomeren, die vorrangig mit der funktionellen Gruppe des difunktionellen Monomers, die eine höhere Reaktivität hat, copolymerisiert, wobei ein erster Block entsteht; und
- einem zweiten ethylenisch ungesättigten Monomer oder zweiten ethylenisch ungesättigten Monomeren, die wenigstens eine Carbonsäuregruppe enthalten und mit der funktionellen Gruppe des difunktionellen Monomers, die eine geringere Reaktivität hat, copolymerisiert, wobei ein zweiter Block entsteht, wobei der erste Block hydrophober ist als der zweite Block und das Copolymer sowohl hydrophobe als auch hydrophile Blöcke aufweist, die wenigstens zwei verschiedene Glasübergangstemperaturen aufweisen, wobei

es sich bei dem polyfunktionellen Monomer um Allylmethacrylat handelt, die ersten ethylenisch ungesättigten Monomere ein Gemisch aus n-Butylacrylat und (Meth)acrylsäure sind und das bzw. die zweiten ethylenisch ungesättigten Monomere aus der Gruppe ausgewählt sind, die aus Acrylsäure, Methacrylsäure und Gemischen davon besteht.

**2.** Copolymer gemäß Anspruch 1, wobei:

das polyfunktionelle Monomer 0,005 bis 2 Molprozent der Gesamtmonomere ausmacht;
die ersten ethylenisch ungesättigten Monomere 5 bis 95 Molprozent der Gesamtmonomere ausmachen; und
das bzw. die zweiten ethylenisch ungesättigten Monomere 5 bis 70 Molprozent der Gesamtmonomere ausmachen.

**3.** Copolymer gemäß Anspruch 1, wobei:

das polyfunktionelle Monomer 0,1 bis 1,5 Molprozent der Gesamtmonomere ausmacht;
die ersten ethylenisch ungesättigten Monomere 5 bis 50 Molprozent der Gesamtmonomere ausmachen; und
das bzw. die zweiten ethylenisch ungesättigten Monomere 10 bis 70 Molprozent der Gesamtmonomere ausmachen.

**4.** Copolymer gemäß Anspruch 1, wobei das Copolymer ein Molekulargewicht von weniger als 1 000 000 hat.

**5.** Copolymer gemäß Anspruch 1, wobei der erste Block ein Molekulargewicht von 10 000 bis 100 000 hat.

**6.** Copolymer gemäß Anspruch 1, wobei der zweite Block ein Molekulargewicht von 1000 bis 100 000 hat.

**7.** Copolymer gemäß Anspruch 1, wobei der erste Block eine Glasübergangstemperatur hat, die niedriger ist als die Glasübergangstemperatur des zweiten Blocks.

**8.** Copolymer gemäß Anspruch 1, wobei die ersten ethylenisch ungesättigten Monomere ein Gemisch aus n-Butylacrylat und Methacrylsäure sind.

**9.** Copolymer gemäß Anspruch 8, wobei das Säuremonomer 50 Molprozent oder weniger des Monomergemischs ausmacht.

**10.** Copolymer gemäß Anspruch 1, wobei die zweiten ethylenisch ungesättigten Monomere ein Gemisch aus Acrylsäure und Methacrylsäure sind.

**11.** Copolymer gemäß Anspruch 1, wobei die ersten ethylenisch ungesättigten Monomere ein Gemisch aus n-Butylacrylat und Methacrylsäure sind, die zweiten ethylenisch ungesättigten Monomere ein Gemisch aus Acrylsäure und Methacrylsäure sind und die Säuremonomere 50 bis 70 Molprozent der Gesamtmonomere ausmachen.

**12.** Copolymer gemäß Anspruch 1, wobei der erste Block eine Glasübergangstemperatur von 30 °C oder weniger hat und der zweite Block eine Glasübergangstemperatur von mehr als 30 °C hat.

**13.** Copolymer gemäß Anspruch 1, wobei der erste Block eine Glasübergangstemperatur von 0 °C oder weniger hat und der zweite Block eine Glasübergangstemperatur von mehr als 0 °C hat.

**14.** Copolymer gemäß Anspruch 1, wobei der zweite Block neutralisiert ist.

**15.** Haarstyling-Zusammensetzung, die 0,01 bis 20 Gew.-% des filmbildenden verzweigten Blockcopolymers gemäß einem der Ansprüche 1 bis 14 umfasst.

**16.** Haarstyling-Zusammensetzung gemäß Anspruch 15, die weiterhin wenigstens eines der folgenden Materialien umfasst:

0 bis 25 Gew.-% eines Emulgators;
0,05 bis 99% Lösungsmittel;
0,05 bis 10% Rheologiemodifikatoren;
0,05 bis 5% Neutralisationsmittel;
0 bis 60 Gew.-% eines flüssigen Treibmittels oder Gases; und
0 bis 1% eines Tensids.

**17.** Verfahren zur Herstellung einer Haarstyling-Zusammensetzung, wobei das Verfahren das Herstellen des filmbildenden verzweigten Blockcopolymers gemäß einem der Ansprüche 1 bis 14 umfasst.

**18.** Verfahren gemäß Anspruch 17, wobei die Herstellung des Copolymers Folgendes umfasst:

Vorlegen eines Lösungsmittels, des polyfunktionellen Monomers, der ersten ethylenisch ungesättigten Monomere und eines Starters in einem Reaktionsgefäß;
Umsetzen der Monomere unter Bildung eines ersten Blocks;
Hinzufügen des zweiten bzw. der zweiten ethylenisch ungesättigten Monomere; und
Umsetzen der Monomere unter Bildung eines zweiten Blocks und eines Copolymers, das sowohl hydrophobe als auch hydrophile Gruppen und wenigstens zwei Glasübergangstemperaturen aufweist.

**19.** Verfahren gemäß Anspruch 18, wobei der Starter aus der Gruppe ausgewählt ist, die aus Startern des Azotyps und Startern des Peroxotyps besteht.

**20.** Verfahren gemäß Anspruch 19, wobei der Starter ein Starter des Azotyps ist, der aus der Gruppe ausgewählt ist, die aus Azobis(dimethylvaleronitril), Azobis(isobutyronitril), Azobis(methylbutyronitril) und Kombinationen davon besteht.

**21.** Verfahren gemäß Anspruch 19, wobei der Starter ein Starter des Peroxotyps ist, der aus der Gruppe ausgewählt ist, die aus Di-t-butylperoxid, t-Butylcumylperoxid, t-Butylperoxypivalat, Laurylperoxid, Cumenhydroperoxid, Ethylhexylperoxodicarbonat, Diisopropylperoxydicarbonat, 4-(t-Butylperoxylperoxycarbonyl)-3-hexyl-6-7-(t-butylperoxycarbonyl)heptylcyclohexen, Cumenhydroperoxid und t-Butylperoxyneodecanoat, t-Butylhydroperoxid, Benzoylperoxid und Kombinationen davon besteht.

**22.** Verfahren gemäß Anspruch 21, wobei es sich bei dem Starter um t-Butylperoxypivalat handelt.

**23.** Verfahren gemäß Anspruch 19, wobei der Starter in einer Konzentration von 0,005 bis 1 Molprozent der Gesamtmonomere vorliegt.

**24.** Verfahren gemäß Anspruch 18, wobei das Lösungsmittel aus der Gruppe ausgewählt ist, die aus Wasser, Kohlenwasserstoffen, Alkoholen, Ethern, Estern, aromatischen Lösungsmitteln, Glycolen, Glycolethern, Glycolestern und Kombinationen davon besteht.

**25.** Verfahren gemäß Anspruch 24, wobei das Lösungsmittel auch Wasser umfasst.

**26.** Verfahren gemäß Anspruch 24, wobei das Lösungsmittel aus der Gruppe ausgewählt ist, die aus Wasser, Ethylalkohol, Isopropylalkohol, t-Butylalkohol, Ethylacetat, Methylacetat, Butylacetat, Benzol, Toluol, Methylenchlorid, Hexan, Cyclohexan, Lösungsbenzin und Kombinationen davon besteht.

**27.** Verfahren gemäß Anspruch 24, wobei es sich bei dem Lösungsmittel um Isopropylalkohol und Wasser handelt.

**28.** Verfahren gemäß Anspruch 18, wobei das Copolymer in demselben Reaktionsgefäß hergestellt wird.

**29.** Verfahren gemäß Anspruch 18, das weiterhin das Neutralisieren des Copolymers in einer solchen Weise, dass

zwischen 0,1 und 100 Prozent der Carbonsäuregruppen neutralisiert werden, umfasst.

**30.** Verwendung des filmbildenden verzweigten Blockcopolymers gemäß einem der Ansprüche 1 bis 14

- als Additive für die Zubereitung von Haarfestigerpräparaten, die aus Aerosol- und Nichtaerosol-Haarspray, Haarlack, Gel, Sprühgel, Schaum, Stylingcremes und Haarglättungsmittel ausgewählt sind;
- für die Herstellung von Shampoos, Haarkuren, Spülungen, Flüssigseife, Seifenstücken, Waschmitteln, Reinigern und Raumdeodorantien;
- als Additive für die Zubereitung von Haar- und Hautcremes, Lotionen, Pomaden und Salben, topisch applizierten Cremes, Hautschutzfilmen, Enthaarungsmitteln, Rasiercremes, Hand- und Körperlotionen, Wimperntuschen und Sonnenschutzmitteln;
- als Additiv in Nagelpflegepräparaten, die aus Nagellack auf Wasserbasis, Nagelhärter und Nagelschutz ausgewählt sind;
- für die Zubereitung von pharmazeutischen Präparaten, die aus Cremes, Pomaden, Gelen, Zahnpasta, Tabletten, Gelkapseln, Einlaufflüssigkeiten, Brechmitteln, Suppositorien, geschäumten Fungizidpräparaten, Wirkstoffabgabezusammensetzungen zur Verabreichung von transdermal aktiven Bestandteilen auf oder durch die Haut, Augentropfen, Aknepräparaten und topischen Schmerzmitteln ausgewählt sind;
- als Additive in Kosmetikanwendungen, Körperimplantaten, Beschichtungen für Katheter, Kanülen, Antitranspirant- und Deodorantpräparaten, Beschichtungen für medizinische Geräte, Handschuhen, ablösbaren Schutzbeschichtungen und Wundverbänden; und
- für die Zubereitung von Tinten, abwaschbaren Schutzbeschichtungen für Textilien, Stoffe und Metallstrippern.

## Revendications

**1.** Copolymère à blocs filmogène formé en présence d'un initiateur de radicaux libres à partir:

- d'un monomère polyfonctionnel ayant deux groupes fonctionnels, la réactivité de l'un des groupes fonctionnels étant supérieure à celle de l'autre groupe fonctionnel;
- de premiers monomères éthyléniquement insaturés copolymérisant préférentiellement avec le groupe fonctionnel du monomère difonctionnel ayant une réactivité plus élevée pour former un premier bloc; et
- d'un second ou de seconds monomères éthyléniquement insaturés contenant au moins un groupe acide carboxylique et copolymérisant avec le groupe fonctionnel du monomère difonctionnel ayant une réactivité plus basse pour former un second bloc, dans lequel le premier bloc est plus hydrophobe que le second bloc, et le copolymère a des blocs hydrophobes comme hydrophiles qui ont au moins deux températures de transition vitreuse différentes, dans lequel

le monomère polyfonctionnel est le méthacrylate d'allyle, les premiers monomères éthyléniquement insaturés sont un mélange d'acrylate de n-butyle et d'acide (méth)acrylique, et le second ou les seconds monomères éthyléniquement insaturés sont choisis dans le groupe consistant en l'acide acrylique, l'acide méthacrylique et des mélanges de ceux-ci.

**2.** Copolymère selon la revendication 1, dans lequel:

le monomère polyfonctionnel comprend 0,005 à 2 pour cent molaires des monomères totaux;
les premiers monomères éthyléniquement insaturés comprennent 5 à 95 pour cent molaires des monomères totaux; et
le second ou les seconds monomères éthyléniquement insaturés comprennent 5 à 70 pour cent molaires des monomères totaux.

**3.** Copolymère selon la revendication 1, dans lequel:

le monomère polyfonctionnel comprend 0,1 à 1,5 pour cent molaires des monomères totaux;
les premiers monomères éthyléniquement insaturés comprennent 5 à 50 pour cent molaires des monomères totaux; et
le second ou les seconds monomères éthyléniquement insaturés comprennent 10 à 70 pour cent molaires des monomères totaux.

4. Copolymère selon la revendication 1, dans lequel le copolymère a un poids moléculaire de moins de 1000 000.

5. Copolymère selon la revendication 1, dans lequel le premier bloc a un poids moléculaire de moins de 10 000 à 100 000.

6. Copolymère selon la revendication 1, dans lequel le second bloc a un poids moléculaire de moins de 1000 à 100 000.

7. Copolymère selon la revendication 1, dans lequel ledit premier bloc a une température de transition vitreuse inférieure à la température de transition vitreuse du second bloc.

8. Copolymère selon la revendication 1, dans lequel les premiers monomères éthyléniquement insaturés sont un mélange d'acrylate de n-butyle et d'acide méthacrylique.

9. Copolymère selon la revendication 8, dans lequel ledit monomère acide comprend au plus 50 pour cent molaires dudit mélange monomère.

10. Copolymère selon la revendication 1, dans lequel les seconds monomères éthyléniquement insaturés sont un mélange de l'acide acrylique et de l'acide méthacrylique.

11. Copolymère selon la revendication 1, dans lequel les premiers monomères éthyléniquement insaturés sont un mélange d'acrylate de n-butyle et d'acide méthacrylique, les seconds monomères éthyléniquement insaturés sont un mélange de l'acide acrylique et de l'acide méthacrylique, et les monomères acide comprennent 50 à 70 pour cent molaires des monomères totaux.

12. Copolymère selon la revendication 1, dans lequel ledit premier bloc a une température de transition vitreuse d'au plus 30 °C et ledit second bloc a une température de transition vitreuse de plus de 30 °C.

13. Copolymère selon la revendication 1, dans lequel ledit premier bloc a une température de transition vitreuse d'au plus 0 °C et ledit second bloc a une température de transition vitreuse de plus de 0 °C.

14. Copolymère selon la revendication 1, dans lequel le second bloc est neutralisé.

15. Composition de mise en forme des cheveux comprenant 0,01 à 20 pour cent en poids du copolymère à blocs ramifié filmogène tel que défini dans l'une quelconque des revendications 1 à 14.

16. Composition de mise en forme des cheveux selon la revendication 15, comprenant en outre au moins un des matériaux suivants: 0 à 25 pour cent en poids d'un émulsifiant;

0,05 à 99 pour cent de solvants;
0,05 à 10 pour cent de modificateurs de rhéologie;
0,05 à 5 pour cent d'agents neutralisants;
0 à 60 pour cent en poids d'un propulseur liquide ou gaz; et
0 à 1 pour cent d'un tensioactif.

17. Procédé pour préparer une composition de mise en forme des cheveux, le procédé comprenant l'étape consistant à préparer le copolymère à blocs ramifié filmogène tel que défini dans l'une quelconque des revendications 1 à 14.

18. Procédé selon la revendication 17, dans lequel la préparation du copolymère comprend les étapes consistant à:

placer dans une chambre de réaction un solvant, le monomère polyfonctionnel, les premiers monomères éthyléniquement insaturés et un initiateur;
faire réagir les monomères pour former un premier bloc;
ajouter le second ou les seconds monomères éthyléniquement insaturés; et
faire réagir les monomères pour former un second bloc et un copolymère ayant des groupes hydrophobes comme hydrophiles et au moins deux températures de transition vitreuse.

19. Procédé selon la revendication 18, dans lequel l'initiateur est choisi dans le groupe consistant en des initiateurs de type azo et des initiateurs de type peroxo.

**20.** Procédé selon la revendication 19, dans lequel l'initiateur est un initiateur de type azo choisi dans le groupe consistant en azo-bis-diméthylvaléronitrile, azo-bis-isobutyronitrile, azo-bis-méthylbutyronitrile et des combinaisons de ceux-ci.

**21.** Procédé selon la revendication 19, dans lequel l'initiateur est un initiateur de type peroxo choisi dans le groupe consistant en peroxyde de di-t-butyle, peroxyde de t-butylcumyle, peroxypivalate de t-butyle, peroxyde de lauryle, hydroperoxyde de cumène, peroxodicarbonate d'éthylhexyle, peroxodicarbonate de diisopropyle, 4-(t-butylperoxyperoxycarbonyl)-3-hexyl-6-7-(t-butylperoxycarbonyl)heptylcyclohexène, hydroperoxyde de cumène et peroxynéodécanoate de t-butyle, hydroperoxyde de t-butyle, peroxyde de benzoyle et des combinaisons de ceux-ci.

**22.** Procédé selon la revendication 21, dans lequel l'initiateur est le peroxypivalate de t-butyle.

**23.** Procédé selon la revendication 19, dans lequel l'initiateur est présent à une concentration de 0,005 à 1 pour cent molaire des monomères totaux.

**24.** Procédé selon la revendication 18, dans lequel le solvant est choisi dans le groupe consistant en eau, hydrocarbures, alcools, éthers, esters, solvants aromatiques, glycols, éthers de glycol, esters de glycol et des combinaisons de ceux-ci.

**25.** Procédé selon la revendication 24, dans lequel le solvant comprend aussi de l'eau.

**26.** Procédé selon la revendication 24, dans lequel le solvant est choisi dans le groupe consistant en eau, alcool d'éthyle, alcool d'isopropyle, alcool de t-butyle, acétate d'éthyle, acétate de méthyle, acétate de butyle, benzène, toluène, chlorure de méthylène, hexane, cyclohexane, essences minérales et des combinaisons de ceux-ci.

**27.** Procédé selon la revendication 24, dans lequel le solvant est l'alcool d'isopropyle et l'eau.

**28.** Procédé selon la revendication 18, dans lequel le copolymère est produit dans la même chambre de réaction.

**29.** Procédé selon la revendication 18, comprenant en outre l'étape consistant à neutraliser le copolymère de manière qu'entre 0,1 et 100 pour cent des groupes acide carboxylique sont neutralisés.

**30.** Utilisation du copolymère à blocs ramifié filmogène tel que défini dans l'une quelconque des revendications 1 à 14

- comme additifs pour la formulation de préparations fixatrices de cheveux choisies parmi un spray capillaire aérosol ou non aérosol, laque capillaire, gel, gel vaporisé, mousse, crèmes coiffantes et défrisages;
- pour la préparation de shampooings, revitalisants, après-shampooings, savon liquide, pains de savon, détergents, produits nettoyants et désodorisants pour locaux;
- comme additifs pour la formulation de crèmes pour les cheveux et pour la peau, lotions, pommades et onguents; crèmes pour application topique, films protecteurs pour la peau, dépilatoires, crèmes à raser les poiles, lotions pour les mains et pour le corps, mascaras et écrans solaires;
- comme additif dans des formulations de soins pour les ongles choisies parmi vernis à ongles à base aqueuse, durcisseur pour ongles et produit pour protéger les ongles;
- pour la formulation de préparations pharmaceutiques choisies parmi crèmes, pommades, gels, dentifrice, comprimés, capsules de gel, liquides pour lavements, vomitifs, suppositoires, préparations fongicides moussées, compositions d'administration de médicaments pour administrer des principes actifs transdermalement sur ou à travers la peau, collyres, préparations antiacnéiques et analgésiques topiques;
- comme additifs dans des applications cosmétiques, implants de corps, revêtements pour cathéters, canules, préparations antisudorales et déodorantes, revêtement pour dispositifs médicaux, gants, revêtements protecteurs éliminables et pansements pour blessures; et
- dans la formulation d'encres, de revêtements protecteurs lavables pour textiles, tissus, et de démétallisants.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 3954960 A, Valan
- US 3914403 A, Valan
- US 3925542 A, Viout
- US 5196495 A, Chuang
- US 4007005 A, Patel
- US 5565193 A, Midha
- US 5620683 A, Tong
- US 5599524 A, Morawski
- GB 1484053 A
- US 5225456 A
- US 5403894 A
- EP 0398576 A
- US 4130517 A
- WO 9709030 A
- WO 9709031 A
- US 4902499 A


### Non-patent literature cited in the description


- Contemporary Polymer Chemistry. Prentice Hall Publishers, 1990
- **H. Immergut ; J. Brandrup.** Polymer Handbook. Interscience, 1989
- McCutcheon's Detergents and Emulsifiers, 1989 Annual. McCutcheon Division, MC Publishing Co, 1989